# EUROPEAN PATENT SPECIFICATION

(11) **EP 3 160 409 B1**
(45) Date of publication and mention of the grant of the patent: **12.09.2018**
(21) Application number: 15739393.5
(22) Date of filing: 26.06.2015
(51) Int. Cl.: A61F 13/00, A61F 13/10

(54) **COMPRESSION SYSTEMS**
KOMPRESSIONSSYSTEME
SYSTÈMES DE COMPRESSION

(30) Priority: 30.06.2014 GB 201411572
(43) Date of publication of application: 03.05.2017
(73) Proprietor: 3M Innovative Properties Company, St. Paul, MN 55133-3427 (US)
(72) Inventor: HITSCHMANN, Guido, D-41453 Neuss (DE); MOHR, Kay, D-41453 Neuss (DE); BECKER, Anja, D-41453 Neuss (DE); BABIC, Ivana, 20134 Milano (IT); CASOTTO, Federico, I-40122 Bologna (IT)
(74) Representative: Müller, Bruno
(86) International application number: PCT/US2015/037902
(87) International publication number: WO 2016/003790

(56) References cited:
- US-A1- 2005 113 729
- US-A1- 2005 192 524
- US-A1- 2011 125 183
- US-B1- 6 338 723

## Description

### Field

The present invention relates to compression systems, in particular compression systems for applying compression to a portion of a limb of a user for the use in the treatment and/or management of oedema and other venous and lymphatic disorders of a limb, more particularly venous leg ulcers and lymphoedema of a limb.

### Background

Compression therapy is generally prescribed to support an insufficient venous or lymphatic system in returning blood or lymph to the heart. Accordingly compression is generally considered to be the standard treatment for use in the treatment of oedema and other venous and lymphatic disorders e.g. of the lower limbs venous leg ulcers and other clinical conditions, such as lymphoedema. The positive effects of compression therapy on venous lymph return as well as on the healing of chronic venous (leg) ulcers are well documented in the medical literature.

Compression bandages and stockings are the most common compression systems used for compression therapy. Compression stockings however often do not provide the desired therapeutic compressive pressure, since such stockings generally need to be quite elastic so that one can pull them on and off. Compression bandages, being made of materials having much lower elastic (or even in some cases nearly non-elastic) characteristics, are typically much more effective in supporting the muscle pump to return venous blood to the heart and thus in compression therapy compared to compression stockings. Compression bandages however typically need to be applied by a well-trained professional in order to achieve the desired and/or needed fit as well as pressure profile, and such bandages typically need to be reapplied frequently due to change (e.g. reduction) in the volume of the limb (e.g. as a result of oedema reduction).

A large number of other compression systems have been proposed. A number of these can be generically described as to include a garment to be wrapped around a limb and a closure mechanism to secure the garment around the limb (e.g. see Us 2011/125183, US 6338723 US 3,538,194, US 3,856,008, US 3,845,769, US 5,653,244, WO 01/72250, US 2002/0062096, US 2003/195449, US 2005/0192524, WO 2006/048619). Although quite of number of non-bandage-type and non-stocking-type compression systems have been proposed, only a few have been commercialized; examples include products marketed under the trade designation FARROWWRAP, JUXTA-FIT and JUXTA-CURES. Notably these commercial products although given as alternatives to typical compression bandaging systems include multiple band-like elements for winding and wrapping (e.g. overlapping and/or cross winding/wrapping).

### Summary of Invention

There is an ongoing need for non-bandage and non-stocking type compression devices that are easy to use and to apply, desirably by non-trained personnel or even the patient, while at the same time facilitating the provision of the desired and/or needed proper fit, which in turns facilitates the provision of desirable uniformity of compression.

In one aspect of the present invention there is provided a compression system for applying compression to a limb of a user comprising a sleeve for substantially covering a portion of the limb of a user, wherein the sleeve has an outer surface, an inner surface, an upper edge, a lower edge and two lateral side edges, wherein in the transverse direction from the first lateral side edge to the second lateral side edge the sleeve comprises a first lateral side region, a central region and a second lateral side region, the first lateral side region of the sleeve being provided with a plurality of fastening bands in series between the upper and lower edges of the sleeve and the second lateral side region being trimmable, and wherein the system further comprises a secondary closure element, said closure element being releasably attachable to the second lateral side region, said closure element further being provided with either a single elongate pair of flaps extending lengthwise to or a plurality of pairs of flaps extending lengthwise in series, said elongate pair of flaps or pairs of flaps, as applicable, each including an exterior flap overlying an interior flap, wherein at least the exterior flap is movable relative to the interior flap such that the exterior flap is capable of performing a hinge movement about a longitudinal or a substantially longitudinal axis-and relative to the interior flap and wherein the opposing inner surfaces of the interior and exterior flaps comprise mechanical fastening elements adapted to releasably engage the inner and outer surfaces, respectively, of at least the second lateral side region of the sleeve and to secure the secondary closure element along the second lateral side region of the sleeve in a clamp-like fashion.

For the sake of clarity, it is to be appreciated that after application of a compression system onto a limb of a user, the transverse direction of the sleeve will also be a circumferential direction. In addition, after plan-conformant application of a compression system onto a limb of a user, the secondary closure element will be secured along the second lateral side edge of the sleeve through the pair(s) of flaps. In this regard it is also to be appreciated that since the second lateral side region is trimmable, in the event the second lateral side region is trimmed during application and/or use of the compression system, the secondary closure element will then be secured to the sleeve along the second lateral side edge generated after trimming. Conversely in the event the second lateral side region is not trimmed, i.e. the compression system is used as is, the secondary closure element will then be secured along the second lateral side edge of the sleeve as originally provided. Finally, after plan-conformant application of a compression system onto a limb of a user, the secondary closure element will also be secured to the sleeve via the fastening bands.

Surprisingly it has been found that by providing a system which in use will provide a sleeve-like garment that can be opened and closed using a series of fastening bands which can be releasably fastened, where the system comprises a sleeve component including a trimmable (second) lateral side region and the fastening bands being (e.g. releasably or fixedly) mounted on or integral with the opposite (first) lateral side region and a secondary closure element including one elongate flap or a plurality pair of flaps in series extending lengthwise, wherein the exterior flap hingedly movement relative to the interior flap and the inner surfaces of the pair(s) of flaps including mechanical fastening elements adapted to releasably engage inner and outer surfaces of the trimmable lateral side region and to secure the secondary closure element along the second lateral side region of the sleeve in a clamp-like fashion (e.g. a V-like or U-like or C-like clamp fashion), it is possible to provide a compression system that is easy to use and apply where the person applying the system can advantageously and quickly provide a good anatomic fit for the particular person wearing the compression system, which in turn facilitates the provision of the needed and/or desired compression.

For example, compression systems described herein can be used as follows: In a first step, the fastening bands can be releasably fastened in conjunction with the secondary closure element, where then the secondary closure element is attached to the sleeve via the bands, desirably at a position which will allow for tightening in a later step. At this point the second lateral side region of the sleeve is free and unattached as well as the flaps of the secondary closure element, where the exterior flap(s) have already been moved or are moved in a hinge- movement away from the interior flap(s) thus exposing the inner surface thereof. In the next steps, the secondary closure element system is positioned along the limb of the user to be treated, the sleeve is wrapped around the limb, in particular moving from the attached side of the sleeve to the unattached side, and then a part of the sleeve, in particular a part of the second lateral side region, is placed over the inner surface of the interior flap(s) on the secondary closure element so that the sleeve covers the limb in a selected manner and fit (e.g. without any folds, wrinkles or gapping) and such that the inner surface of the sleeve releasably engages with the mechanical fastening elements provided on the inner surface of the interior flap(s). It will appreciated that the sleeve can be repeatedly disengaged from and reengaged with the mechanical fastening elements on the interior flap(s) until the desired fitting of the sleeve about the limb is achieved. Once the desired fit of the sleeve about the limb is achieved, if applicable, any excess and unengaged material of the sleeve extending beyond the inner surface of the interior flap(s) (e.g. distal to the central portion of the sleeve) can be trimmed off e.g. with a pair of scissors. Thereafter, the exterior flap(s) can be then moved in a hinge-movement into contact with a part of the outer surface of the sleeve (in particular that part of the sleeve whose inner surface is engaged with the interior flap(s), more particularly that the part of the second lateral side region of the sleeve whose inner surface is engaged with interior flap(s)), so that the mechanical fastening elements provided on the inner surface of the exterior flap(s) releasably engage said part of the outer surface of the sleeve, thus completing the securing of the secondary closure element along the second lateral side edge of the sleeve. At this point, the secondary closure element is attached to the sleeve via the flaps in a clamped configuration onto the respective second lateral side region of the sleeve as well as via the fastening bands to the first lateral side region of the sleeve. Also at this point, desirably the sleeve is sitting in a conformed fit, but not in a tightened fit for providing the ultimate needed and/or desired level of compression. Moreover, after the sizing operation and attachment of the closure element onto the sleeve via the flaps, the fastening bands are favorably unfastened, repositioned so as to tighten the sleeve about the limb of the user and to provide a desired level of compression and then refastened. It should be appreciated that if, as in fact is desired, the volume of the limb is reduced (for example as a result of oedema reduction due to effective compression therapy) to such extent that the level of compression is lower than needed and/or desired, one can re-tighten the compression system or if need be, re-apply the system with further trimming of the sleeve component.

Favorably, the mechanical fastening elements provided on the inner surfaces of the flaps may comprise male fastening elements, in particular male fastening elements selected from the group consisting of hook fasteners, mushroom-shaped fasteners, stem-shaped fasteners, cup-shaped fasteners, T-shaped fasteners, pin-shaped fasteners and mixtures thereof. As mentioned above the fastening elements are selected to engage the inner and outer surfaces of at least the second lateral side region of the sleeve. For a greater freedom in sizing and general use it may be advantageous to select fastening elements to engage in addition the inner and outer surfaces of the second lateral side region, the inner and outer surfaces of the central region. It will be appreciated that the inner and outer surfaces of the respective region(s) of the sleeve may have a structure that can be engaged by mechanical (e.g. male) fastening elements or certain type(s) of mechanical (e.g. male) fastening elements and/or may be provided with a structure that is adapted to be engaged by mechanical (e.g. male) fastening elements or certain type(s) of mechanical (e.g. male) fastening elements (e.g. laminated with a loop engagement material). It will be appreciated that the structure of the inner and outer surfaces of the respective region(s) of the sleeve may be the same or different. Also the mechanical (e.g. male) fastening elements provided on the inner surface of the interior and exterior flaps may be the same or different. It has been found advantageous to provide different mechanical (e.g. male) fastening elements on the interior and exterior flaps.

It has been found advantageous to configure and arrange the inner surface of at least the second lateral side region of the sleeve (in particular the second lateral side region and central region of the sleeve) and the mechanical, in particular male, fastening elements of the inner surface of the interior flap so as to provide sufficient shear strength, e.g. shear strength of at least 2 N/cm² as measured according to EN13780 using a sample width of 5 cm for each material and an overlap of 2 cm, to facilitate the sizing and trimming steps and to minimize slippage of the sleeve material during these steps. Furthermore, it has been found that in order to facilitate repeated operations of attachment, detachment and attachment during the sizing step until a desired fit is achieved, it has been found advantageous that the peel strength between the aforesaid surfaces be relatively low, e.g. less than 0.6 N/cm, more favorably equal to or less than 0.3 N/cm, as measured according to EN 12242 using a sample width of 5 cm for each material. In this regard it has been recognized that low peel strengths are not detrimental in terms of the use of the compression system during compression therapy since the interior flap is positioned behind the sleeve towards the user, and thus any inadvertent detachment during use is virtually excluded.

It has been found advantageous to configure and arrange the outer surface of at least the second lateral side region of the sleeve (in particular the second lateral side region and central region of the sleeve) and the mechanical, in particular male, fastening elements of the inner surface of the exterior flap so as to provide a relatively high shear strength, e.g. shear strength of at least 7 N/cm² as measured according to EN13780 using a sample width of 5 cm for each material and an overlap of 2 cm, to minimize any slippage of the sleeve material during use. Furthermore, it has been found advantageous that the peel strength between the aforesaid surfaces be relatively high, e.g. peel strength at least 0.6 N/cm as measured according to EN 12242 using a sample width of 5 cm for each material, to facilitate secure fastening as well as to minimize inadvertent lifting and de-attachment of the exterior flap during use. Inadvertent lifting and de-attachment of the exterior flap can be further minimized by further providing the closure element with a locking element or a series of locking elements in association the exterior flap or flaps, said locking element(s) and exterior flap(s) being configured and arranged such that in use when the closure element is attached to the sleeve via the flaps, the locking element(s) can be clamped, e.g. mechanically clamped, onto the exterior flap(s), in particular onto the outer surface of the exterior flap(s). In order to facilitate purposeful releasable detachment and re-attachment of the exterior flap by the user or care-giver e.g. in order to re-fit and/or re-apply the compression system, desirably the peel strength is at most 10 N/cm, more desirably at most 5 N/cm.

Favorably, the closure element includes one or more elongate stiffeners extending longitudinally. Desirably, the closure element comprises a spacer fabric and/or a foam (in particular memory foam, more particularly high density memory foam). More desirably, the closure element comprises a multilayer construction comprising at least one layer, in particular two or more layers, of a material selected from the group consisting of a spacer fabrics, foams or combinations thereof. The closure element, excluding any structural elements attached thereto (e.g. flaps, rings, straps), may have a thickness of from 2 mm to 12 mm, inclusive, in particular a thickness from 3 mm to 8 mm, inclusive. In a region free of seams, if applicable stiffeners, and any structural elements attached thereto (e.g. flaps, rings, straps), the closure element favorably has an air permeability equal to or greater than 40 cm/s, more favorably equal to or greater than 80 cm/s, even more favorably equal to or greater than 120 cm/s, most favorably equal to or greater than 160 cm/s, as measured according to ISO 9237:1995 using a test pressure of 200Pa. A test area of 20 cm² is typically measured. It is to be recognized that the units for air permeability are strictly speaking volume/area time, cm³/(cm² sec), and testing devices, such as Air Permeability Tester supplied by TexTest Instruments, Zurich, often display measured air permeability values in unit of cm/s. In addition or alternatively in a region free of seams, and flaps, and, if applicable, free of other added structural elements (such as rings, straps or stiffeners), the closure element favorably has an water vapor transmission rate equal to or greater than 1000 g/ (m² ×24 h), more favorably equal to or greater than 1500 g/ (m² ×24 h), even more favorably equal to or greater than 2000 g/ (m² ×24 h), most favorably equal to or greater than 2500 g/ (m² ×24 h), as measured according to ISO 15106, part 1.

Desirably, the secondary closure element is configured as a tongue. The closure element is favorably configured and arranged relative to the sleeve such that, in use when the closure element is attached to the sleeve via the flaps and the fastening bands are fastened, the closure element is generally centrally positioned adjacent to and extends along the first and second lateral edges of the sleeve, so that the secondary closure element is located between the user and an opening defined between the first and second lateral edges of the sleeve

In normal use of the compression system, i.e. towards the provision of compression, e.g. when the secondary closure element already is attached to the sleeve via the flaps, the fastening bands are favorably pulled so that the first lateral side edge is drawn towards the second lateral edge of the sleeve thereby tightening the sleeve about the limb and then fastened so that the sleeve is restrained about the limb of the user. More favorably in this operation the two lateral edges of the sleeve are drawn towards one another, but do not overlap.

Fastening bands may be either integral with the first lateral side region of the sleeve such that fastening bands extending in substantially the transverse direction of the sleeve out from the first lateral side edge of the sleeve or each fastening band may comprise a proximal end portion, said proximal end portion being releasably or fixedly attached to the first lateral side region of sleeve such that the band extends substantially the transverse direction of the sleeve over first lateral side edge. Having regard to the first alternative, it will be appreciated that the first lateral side edge of the sleeve will be understood to run along the outer edges of the first lateral side region of the sleeve located between fastening bands and, if applicable, next to the uppermost and/or lowermost fastening bands and the boundary between the first lateral side region of the sleeve and the fastening band. Desirably the part of the fastening bands extending beyond the first lateral side edge has a width relative to the transverse direction of the sleeve of at least 3 cm, in particular at least 6 cm. In addition or alternatively, favorably the part of the fastening bands extending beyond the first lateral side edge has a width relative to the transverse direction of the sleeve is at most 25 cm. For those embodiments including proximal end portions, desirably the proximal end portions of the fastening bands are connected and/or integral to one another.

Favorably each fastening band comprises a distal end portion, wherein each band extends in substantially the transverse direction of the sleeve with its distal end portion positioned away from the central portion of the sleeve. It will be appreciated that for those embodiments including fastening bands including both proximal end portions and distal end portions, the fastening may include an inner band portion therebetween. Similarly, for those embodiments including fastening bands that are integral to the sleeve, there may be in inner band portion between the distal end portion and the sleeve portion. Desirably at least the distal end portions of the fastening bands have a height relative to the transverse direction of the sleeve of at least 1 cm, in particular at least 2 cm, more particularly at least 3 cm. In addition or alternatively, favorably at least the distal end portions of the fastening bands have a height relative to the transverse direction of the sleeve of at most 10 cm, in particular at most 8 cm, more particularly at most 6 cm.

Favorably the distal end portions of the fastening bands comprise mechanical fastening elements, in particular male fastening elements, more particularly male fastening elements selected from the group consisting of hook fasteners, mushroom-shaped fasteners, stem-shaped fasteners, cup-shaped fasteners, T-shaped fasteners, pin-shaped fasteners and mixtures thereof.

Fastening bands may be fastened directly onto appropriate complementary engaging structure provided on the secondary closure element, in particular provided onto the outer surface of the secondary closure element. For example, each fastening band typically has a first major surface and a second major surface, the first major surface facing inwardly (towards the wearer) and the second major surface facing outwardly (away from the wearer), mechanical fastening elements of the distal end portion may be favorably provided on the first major surface at the distal end portion. Desirably the outer surface of the secondary closure element has a structure or is provided with a structure that is adapted to be engaged by the mechanical fastening elements on the first major surface of the distal end portion of the fastening bands. In such embodiments, favorably the pair of flaps or pairs of flaps, as applicable, extend lengthwise to one side of the engaging structure for the fastening bands. When the secondary closure element is attached via flaps to the second lateral side edge, desirably the engaging structure for the fastening band is located distal to the second lateral side edge. In favorable embodiments, the axis for hinged movement of the exterior flap or each of the exterior flaps, as applicable, is positioned proximal to the engaging structure for the fastening bands. In this manner when the exterior flap(s) are moved in a hinge-movement there are moved away from the interior flap(s) towards said engaging structure, thus facilitating subsequent positioning and attachment of the sleeve while minimizing conflict and/or interference with the engaging structure.

Or alternatively and favorably, the secondary closure element, in particular the outer surface thereof, may be provided with a plurality of rings in series such that when the closure element is attached to the sleeve, there is a ring located opposite to a fastening band. In such embodiments, favorably the pair of flaps or pairs of flaps, as applicable, extend lengthwise to one side of the series of rings. Again to facilitate positioning and attachment of the sleeve while minimizing conflict and/or interference with the rings, the axis for hinged movement of the exterior flap or each of the exterior flaps, as applicable, is positioned proximal to the series of rings. In addition or alternatively, the series of rings of such embodiments are configured and arranged such that in use when the closure element is attached to the sleeve along the second lateral side edge of the sleeve, the series of rings is located distal to the second lateral side edge. For favorable embodiments including rings, desirably the fastening bands and rings, are configured and arranged such that, in use, the bands can be passed through the rings then turned back on themselves, such that the first lateral side edge of the sleeve is drawn towards the rings and so that the sleeve is tightened about the limb of the user and then fastened so that the sleeve is restrained about the limb of the user.

As mentioned above, each fastening band typically has a first major surface and a second major surface, the first major surface facing inwardly and the second major surface facing outwardly, and favorably the mechanical fastening elements of the distal end portion are provided on the second major surface at the distal end portion. Desirably the outer surface of at least first lateral side portion of the sleeve has a structure or is provided with a structure that is adapted to be engaged by the mechanical fastening elements on the second major surface of the distal end portion of the fastening bands. In addition or alternatively for those embodiments including fastening bands with proximal end portions, favorably proximal end portions are attached to the outer surface of the first lateral side region of the sleeve and the second major surface at the proximal end portion of the band has a structure or is provided with a structure that is adapted to be engaged by the mechanical fastening elements on the second major surface of the distal end portion of the fastening bands.

It will be appreciated that in the event the outer surface of at least the first lateral side region of the sleeve has or is provided with a structure adapted to be engaged by the mechanical fastening (e.g. male) elements provided on the second major surface of distal end portions of the fastening bands, it may be advantageous to use mechanical (e.g. male) fastening elements on both the second major surface of the distal end portion of the fastening bands and the inner surface of the exterior flap(s), which are the same or equivalent. In addition or alternatively, favorably the outer surface of the first and second lateral side regions, in particular the outer surface of the central region and the first and second lateral side regions, has a structure or is provided with a structure that is adapted to be engaged by both the mechanical fastening elements provided on the inner surface of the exterior flap(s) and on the second major surface of the distal end portion of the fastening bands. For those embodiments where all three regions of the sleeve are structurally the same, so that that there is no discernible boundary between the three regions, it will be generally understood that in regard to the continuous joint region of the three, that second lateral side region will be generally understood to be the third of joint region adjacent to the second lateral side edge, the first lateral side region the third of the joint region adjacent to the first lateral side edge and the central region the third of the joint-region located there between.

In order to provide a desirable visual indication facilitating an assessment towards the extent of extension and the provision of uniform compression, it has been found advantageous to provide a strap for each ring, one end of the strap being connected to the closure element (in particular to the outer surface thereof) and the other end bearing the ring, said strap extending between the closure element and the ring in substantially the transverse direction relative to the sleeve and, at least a portion of said strap being expandable in at least the transverse direction, wherein said expandable portion comprises a material having elasticity in at least the transverse direction and is configured and arranged, such that when the expandable portion is in its non-expanded state there is exteriorly a loop of material rising outwardly and when, in use when the closure element is attached to the sleeve and tension is provided in the transverse direction of the sleeve, the expandable portion expands in the transverse direction and the loop flattens.

In accordance with ASTM D4848-98 (2012) and BS EN 14704-1:2005 elasticity is that property of a material by virtue of which it tends to recover its original size and shape immediately after removal of the force causing deformation.

For those embodiments including expandable strap portions, when there is no extension or only partial extension of the expandable strap portion, the outwardly facing loop will be fully raised or only partly flattened, and thus visible as such, and when there is full extension of the expandable strap portion the outwardly facing loop will disappear (i.e. it will flatten to such an extent there is no longer a loop of material rising outwardly). Such a visual indication is advantageous during the application because once the loop fully flattens out (and thus disappears) there is full extension and thus an indication towards sufficient compression. Moreover, by providing such a loop-indicating configuration in the straps of the plurality of rings that are provided in series between the upper and lower edges of the system, i.e. the upper and lower edges of the secondary closure element, it is possible to have a visual indication towards extent of extension and thus compressive fit over respective height of the sleeve between its upper and lower edges, so that if desired and/or needed, the extent of tightening of an individual fastening band can be adjusted, thus facilitating the provision of a desirable compressive fit over the portion of the limb of a user covered by the compression system and its sleeve and thus in turn facilitating uniformity of compression. The loop-indicating configuration is also favorably useful while the user is wearing the compression system. For example, if, as in fact is desired, the volume of the limb is reduced for example as a result of oedema reduction due to effective compression therapy, the extent of tension on the system and on the expandable strap-portion will be reduced and the previously flattened loop will then noticeably pucker outwardly forming a fully raised or somewhat flattened loop, depending on the extent of reduced tension and thus providing an indication that the system should be re-tightened and/or re-applied.

The above summary of the present invention is not intended to describe each disclosed embodiment or every implementation of the present invention. The description that follows more particularly exemplifies illustrative embodiments. Also further embodiments are described in dependent claims. In several places throughout the application, guidance is provided through lists of examples, which examples can be used individually and in various combinations. In each instance, the recited list serves only as a representative group and should not be interpreted as an exclusive list.

### Brief Description of Drawings

The invention will now be described with reference to the accompanying drawings in which:

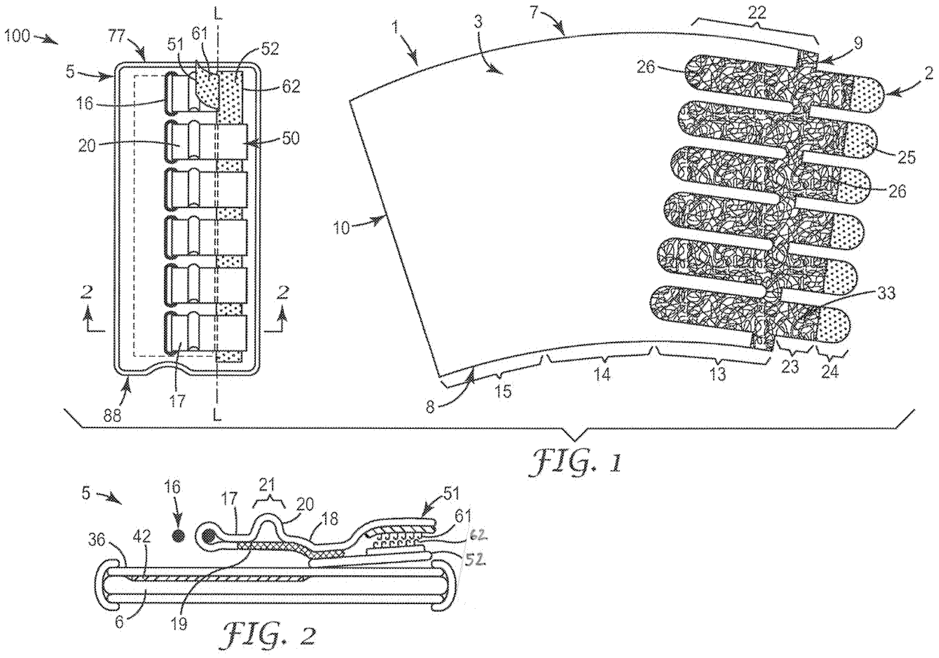

Figure 1 represents a top view of an exemplary embodiment of a compression system in accordance with the invention described herein, while Figure 2 shows a cross-sectional view of a part (i.e. the secondary closure element) of the exemplary embodiment depicted in Figure 1.

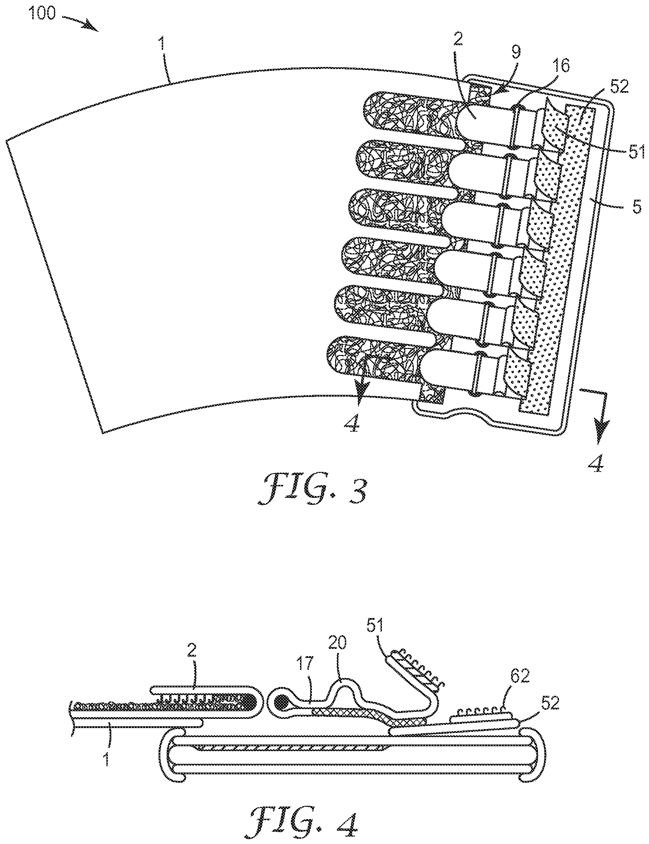

Figure 3 represents a top view of the exemplary embodiment depicted in Figure 1 shown in a configuration prior to use wherein the sleeve and secondary closure element are attached via the fastening bands, while Figure 4 shows a partial cross-sectional view of the exemplary embodiment in the configuration depicted in Figure 3.

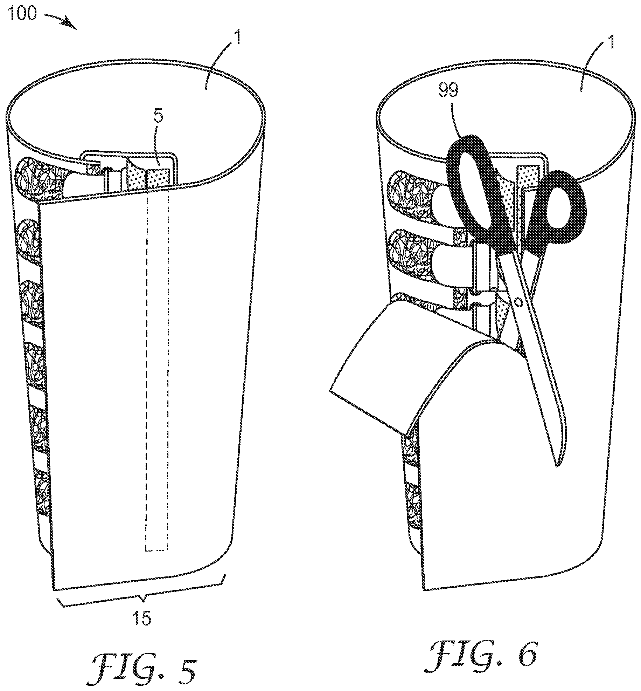

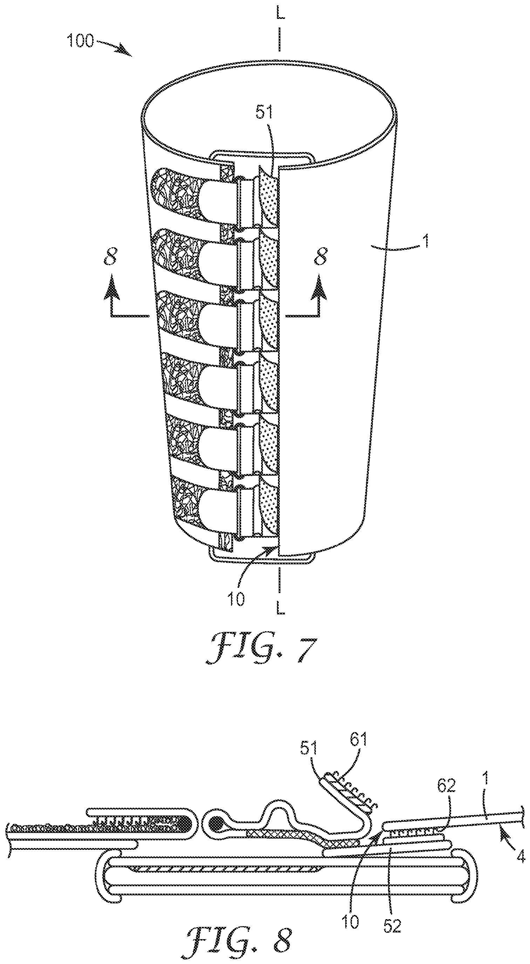

Figures 5 to 7 represent perspective, front views of the exemplary embodiment depicted in Figure 1, shown in configurations corresponding to wrapping the embodiment about a limb a user (limb not shown) during sizing (Figure 5), during trimming (Figure 6) and after trimming (Figure 7), while Figure 8 shows a partial cross-sectional view of the exemplary embodiment in the configuration depicted in Figure 7.

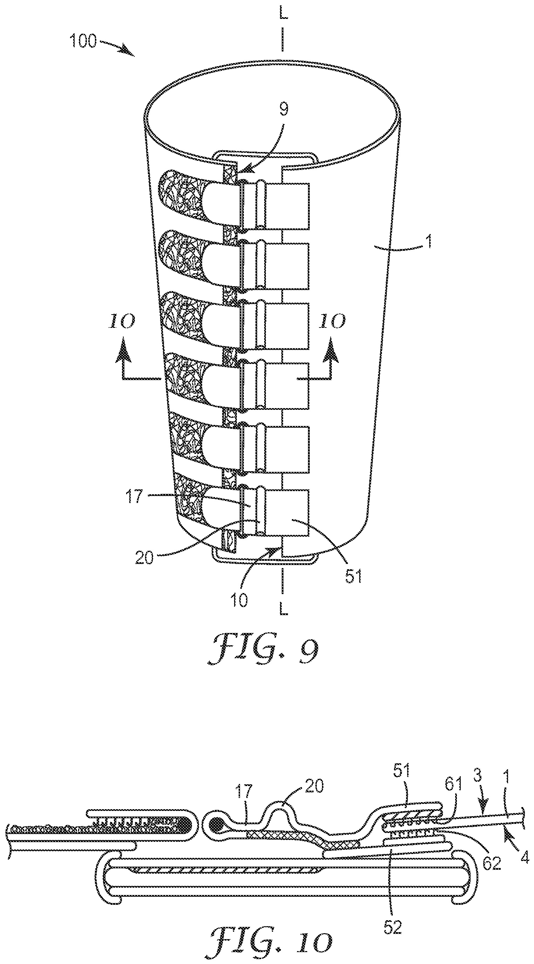

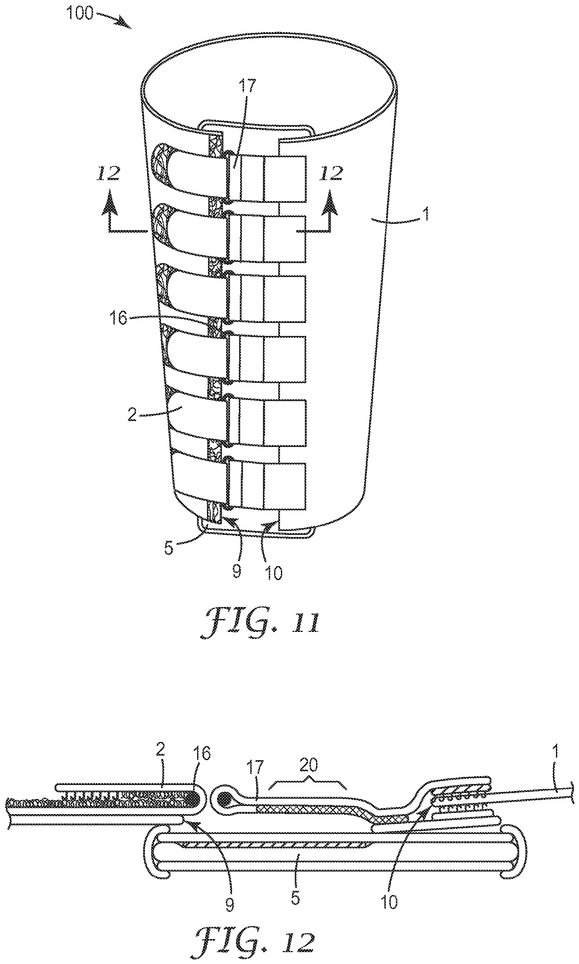

Figures 9 and 11 represent perspective, front views of the exemplary embodiment depicted in Figure 1 shown in configurations corresponding to the embodiment being about a limb of a user (limb not shown) with the sleeve and secondary closure element being attached via the flaps and the fastening bands, where the sleeve is not tightened (Figure 9) and where the sleeve is tightened (Figure 11), while Figures 10 and 12 show partial cross-sectional views of the exemplary embodiment in the configurations depicted in Figures 9 and 11, respectively.

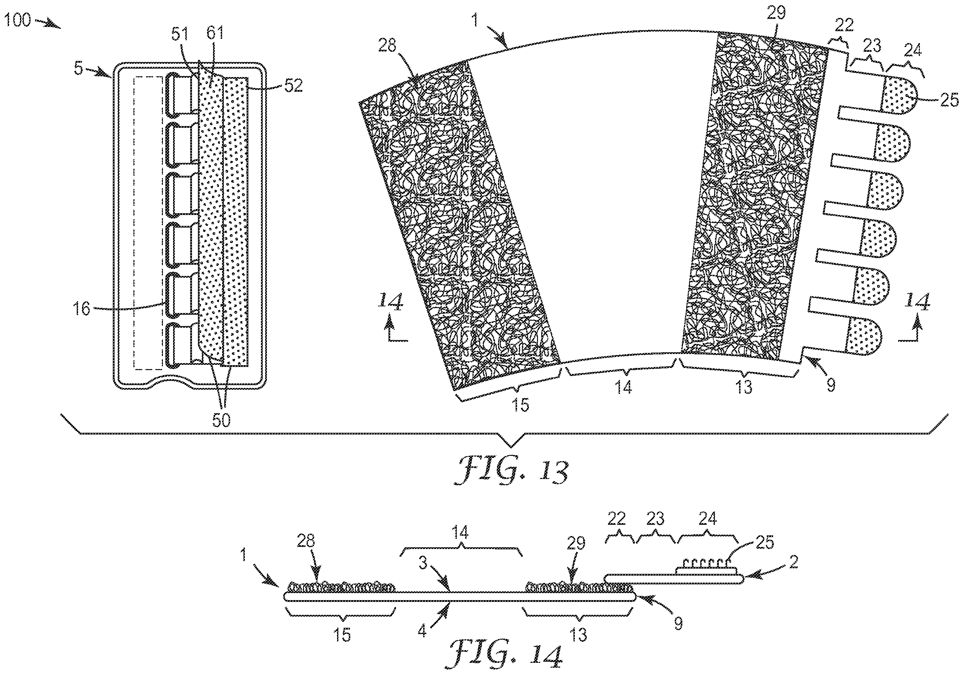

Figure 13 represents a top view of another exemplary embodiment of compression system in accordance with the invention described herein, while Figure 14 shows a cross-sectional view of a part (i.e. the sleeve element) of the exemplary embodiment depicted in Figure 13.

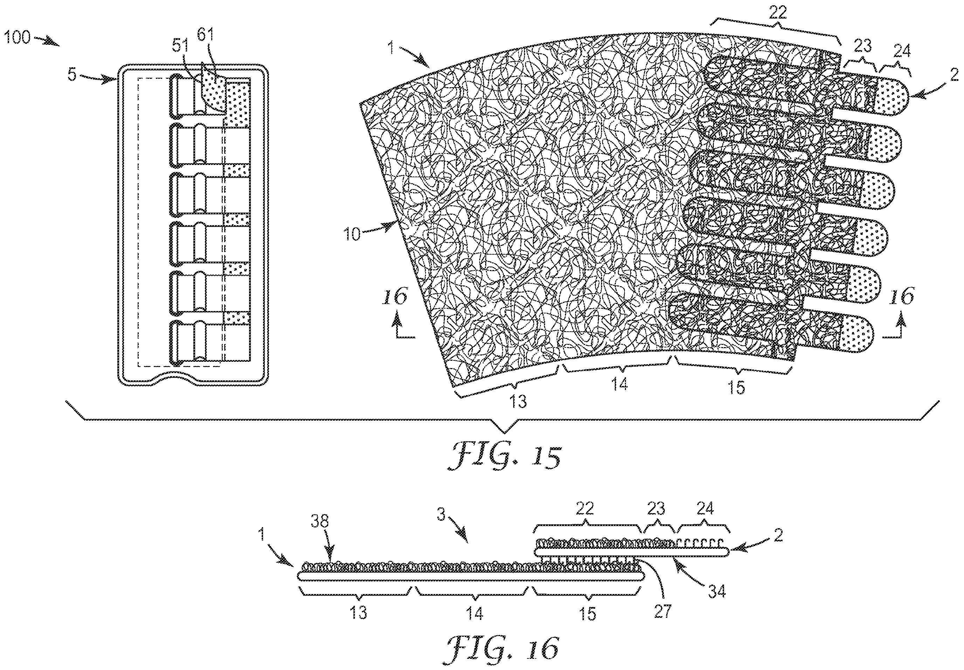

Figure 15 represents a top view of an additional exemplary embodiment of a compression system in accordance with the invention described herein, while Figure 16 shows a cross-sectional view of a part (i.e. the sleeve element) of the exemplary embodiment depicted in Figure 15.

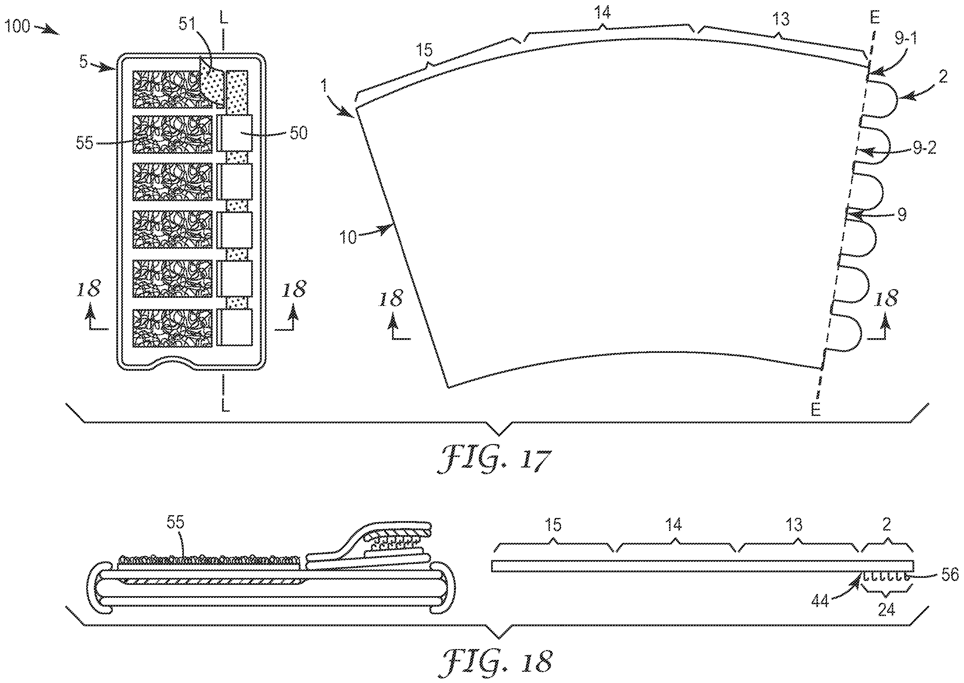

Figure 17 represents a top view of a further exemplary embodiment of a compression system in accordance with the invention described herein, while Figure 18 shows a cross-sectional view of the exemplary embodiment depicted in Figure 17.

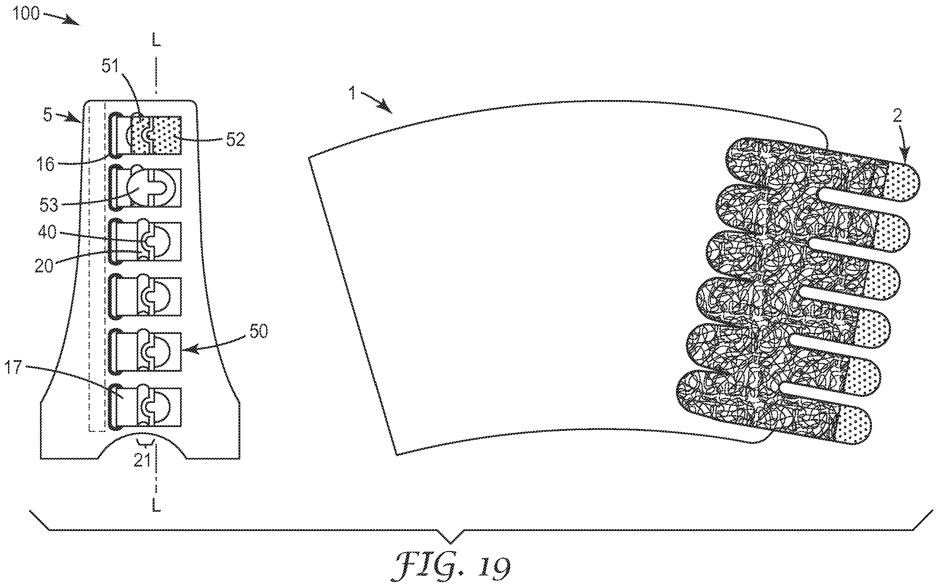

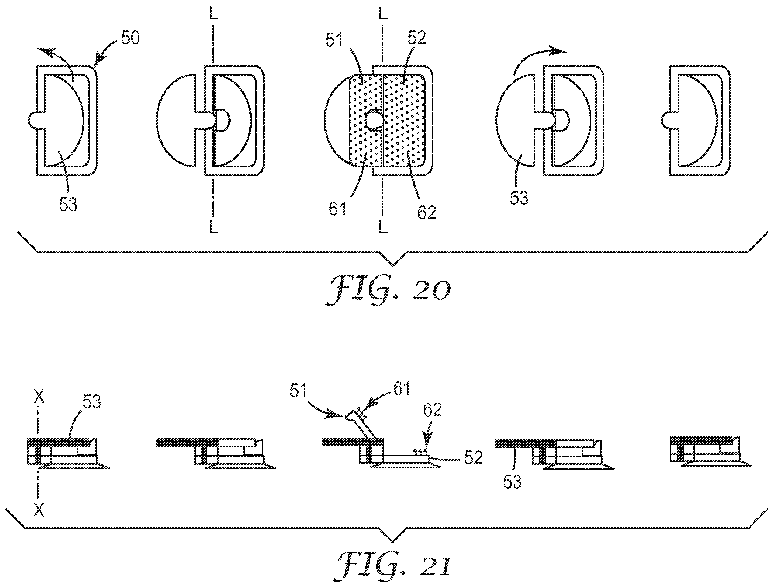

Figure 19 represents a top view of yet a further exemplary embodiment of a compression system in accordance with the invention described herein, while Figures 20 and 21 show top and cross-sectional views of one of the pairs of flaps of the exemplary embodiment depicted in Figure 19 in a series of configurations from locked to hinged open back to locked.

In the description that follows, unless expressly stated otherwise, terms such as 'top', 'bottom', 'above', 'below', etc, refer only to features as shown in the Figures, and no restriction as to orientation of use, etc, is intended. Not all Figures are to the same scale.

### Detailed Description

It is to be understood that the present invention covers all combinations of particular, suitable, desirable, favorable, advantageous and preferred aspects of the invention described herein.

Figure 1 shows a top view of the exterior of an exemplary embodiment of a compression system (100) comprising a sleeve (1) for substantially covering a portion of the limb of a user and a secondary closure element (5), while Figure 2 shows a cross-sectional view of the secondary closure element. The sleeve includes an outer surface (3), an inner surface (4; not numbered on Figure 1), an upper edge (7) and a lower edge (8). When the device is in use on the limb, typically the inner surface (4) is located towards the wearer/user (in the following the term "inner" will typically refer to something located towards the wearer/user and "outer" away from the wearer/user), while the upper edge is located towards to the torso of the user and the lower edge distant to the torso of the user, and both upper and lower edges, being essentially transverse, will be located essentially circumferentially around the limb after application. As can be appreciated from Figure 1, the sleeve includes two lateral side edges (9, 10) extending from its upper edge to its lower edge. In the transverse direction from the first lateral side edge (9) to the second lateral side edge (10) the sleeve comprises a first lateral side region (13), a central region (14) and a second lateral side region (15). The central region and the second lateral side region may structurally be the same as shown in the exemplary embodiment illustrated in Figure 1. In alternative embodiments, the central region and the second lateral side region may be structurally different, for example as can be seen in the exemplary compression system (100) depicted in Figure 13, where the outer surface (3) of the second lateral side region (15) of the sleeve (1) is provided with, in particular laminated to, an additional material layer (e.g. a loop engagement material) (28) having structure that can engage mechanical fastening elements (61) provided on the exterior flap (51) provided on the secondary closure element (5). See also Figure 14, which provides a cross-sectional view of the sleeve in Figure 13. In other alternative embodiments, the entire outer surface of the sleeve may be provided (e.g. laminated) with a material (e.g. a loop engagement material) having structure that can engage mechanical fastening elements provided on the exterior flap(s) of the secondary closure element. Returning to the exemplary embodiment depicted Figure 1, it will be appreciated that since the central region and second lateral side region are structurally the same, there is no discernible boundary between the two regions. Having regard to the continuous region of the two, i.e. the joint-region, in the following the second lateral side region (15) will be generally understood to be the half of the joint-region that is adjacent to the second lateral side edge (10) while the central region will be generally understood to be the half of the joint-region adjacent to the first lateral side region. The second lateral side region (15) is free of structural elements like fastening bands, rings, engaging structure, etc. and thus is configured and arranged so that it can be trimmed, while the first lateral side region (13) of the sleeve is provided with a plurality of fastening bands (2) in series between the upper and lower edges of the sleeve, the bands being in this exemplary embodiment fixedly attached (e.g. sewn) to the outer surface at the first lateral side region of the sleeve.

Sleeves, when laid out flat for example as shown in Figure 1, may be substantially rectangular, or substantially trapezoidal or irregular in shape. For example, the sleeves in exemplary embodiments depicted herein (e.g. in Figure 1) are substantially trapezoidal in shape. For facilitating an optimal fit onto a part of the limb of a user, the upper edge and/or the lower edge of the sleeve may be favorably slightly curved, in particular the upper edge may be slightly convex and/or the lower edge which is normally positioned distant to the torso of the user, may be either slightly concave or convex. Alternatively or in addition thereto, one or both of the lateral side edges may be slightly curved, in particular slightly convex. This may be facilitating fitting over well-developed calves. In use, when the compression device is applied onto a limb of the user, favorably the sleeve is substantially cylindrical, barrel or truncated-conical in shape. It is to be noted after use and removal of the compression system from the limb of a user that the sleeve when laid out may not be perfectly flat.

Sleeves, in particular at least the central region thereof, favorably comprise a material that is suitable for use in applying compression. Such materials are known in the art, including for example the material used in the compression product marketed under the trade designation JUXTA-CURES or the material used in the "Handgelenk Schiene" (wrist brace/splint) marketed under the trade designation 3M FUTURO. Other materials that may be suitable include spacer fabrics e.g. such as those described in US 5,385,036. Example of suitable spacer fabrics include the spacer fabrics marketed under the trade designation SHR755 D3 and SHR 775 D3 by Gehring-Tricot Corporation, Garden City, NY 11530, USA. Favorably materials used have low flexural rigidity and are stretchable, so that they can readily adapt to the shape of the limb shape, while at the same time not being too easily stretched under tension, so that the desired provision of compression onto the limb can be achieved. More favorably at least the central region of the sleeve (even more favorably at least the central and the second lateral side regions of the sleeve, most favorably the central region as well as the first and second lateral side regions of the sleeve) comprises a material having elasticity and a maximum stretch greater than 0% up to 60% in at least the transverse direction of the sleeve under a load of 10 N per cm width e.g. as measured in accordance with DIN 61632:2009. Herein such a material is referred to as a "short-stretch material". Referring to the textbook Foeldi's Textbook of Lymphology, 2nd Edition, extensibility, in other words elasticity, of compression bandages include short stretch (those having a maximum amount of stretch under a load of 10 N per cm width of up to 60%, i.e. greater than 0% and up to 60%); moderate-stretch (60 to 140%) and long stretch (greater than 140%), where correspondingly non-elastic bandages are understood to have no amount of (i.e. 0%) stretch. For comfort in wearing, such materials are desirably air permeable, more desirably have an air permeability of at least 20 cm/sec, more desirably at least 60 cm/sec, even more desirably at least 100 cm/sec, most desirably at least 200 cm/sec according to test ISO 9237-1995 using at a test pressure of 200Pa and/or a water vapor according to test ISO 9237-1995 using at a test pressure of 200Pa. In addition or alternatively, sleeve materials favorably have an water vapor transmission rate equal to or greater than 1000 g/ (m² ×24 h), more favorably equal to or greater than 1500 g/ (m² ×24 h), even more favorably equal to or greater than 2000 g/ (m² ×24 h), most favorably equal to or greater than 2500 g/ (m² ×24 h), as measured according to ISO 15106, part 1.

Sleeves may be provided with one or more stiffeners extending lengthwise to facilitate maintenance of sleeve shape and/or to improve local pressure distribution, in particular to minimize any tendency towards vertical collapsing or slipping-down of the sleeve, stiffeners may be provided e.g. in the form of wires, bars, grids, or pads having limited width relative to the transverse direction of the sleeve. For example, an elongate stiffener that extends lengthwise between the upper and lower edges of the sleeve could be provided in the first lateral side region, in particular adjacent to the first lateral edge. Stiffeners may be made of e.g. metal or thermoplastic materials including thermoformable thermoplastic materials (such as polypropylene, polyamide, polyester (e.g. 3M SCOTCHCAST ™ Thermoplastic Material 72362)). For stiffeners having a width greater than five millimeters, it may be favorable to provide them with perforations to allow for breathability. For design and/or fixing purposes, stiffeners may be provided within a fabric pocket which is subsequently attached to the appropriate part(s) of the sleeve or alternatively stiffeners may be positioned on the surface of the appropriate part(s) of the sleeve, which are then covered completely with a sheet of fabric that is sewn or laminated onto the respective part(s) of the sleeve.

As mentioned the provision of a trimmable second lateral side region in conjunction with other features of the compression systems described herein facilitates the provision of good and individualized anatomic fit, which in turn facilitates the provision of the needed and/or desired compression for that particular user. Sleeves of compression systems described herein can be provided in different sizes to accommodate the difference in the size of limbs (e.g. arms versus legs) as well as the general difference in sizes of a particular limb. Compression systems described herein are particularly suitable for use on the lower leg including the calf. In regard to the latter considering the length of an adult human lower leg can range from around 20 cm to 50 cm, it could be possible to provide, three height sizes, e.g. short, average and, tall, again aimed to cover 80% of the potential relevant lengths of the potential users. In addition and more significantly, considering the size of an adult human lower leg, including those persons suffering from lymphedema, can range from around 130 to 420 mm in circumference at the ankle and around from 280 to 650 mm in circumference at their widest point, it could be possible to provide compression systems in for example seven standard width sizes, e.g. XS, S; M, L, XL, XXL, XXXL, aimed to cover 80% of the potential relevant circumferential sizes of the potential users while the remaining 20% could be provided for by special order. The application of compression systems described herein where the second lateral side region of the sleeve is configured such that it is trimmable is also advantageous in that it allows for the reduction in the number of standard sizes to cover 80% the potential relevant circumferential sizes of the potential users. For example, instead of providing seven sizes one could provide three sizes corresponding in size to M, XL and XXXL indicated above, where then the width of the sleeve could be readily adjusted by the user or the care-giver applying the compression system onto the limb of the user to smaller sizes, e.g. XL down to L. In addition as mentioned above, in the event that the compression therapy is effective (as it should be) such that the circumference of the limb is significantly reduced, the width of the sleeve can be easily further re-adjusted by the user or the care-giver to even a smaller size, e.g. L to M. In the illustrated exemplary embodiment depicted in Figure 1, the fastening bands are fixedly attached to the first lateral side region In order to facilitate a further degree of freedom or flexible as to sizing, especially having regard to the aforesaid "remaining 20%" that would normally needed to be provided for by special order, in alternative embodiments, the fastening bands may be releasably attachable to the first lateral side region of the sleeve, where then the first lateral side region of the sleeve may also be configured and arranged such that it is trimmable. For such embodiments, it can be envisioned that compression systems described herein may be favorably provided in a system including sleeve material, e.g. in a roll, with one or more sets including an appropriate series of the fastening bands and a secondary closure element.

Returning to the exemplary embodiment of Figure 1, each fastening band comprises a proximal end portion (22) and a distal end portion (24) being connected by an inner band portion (23). In the exemplary embodiment shown here in Figure 1, the proximal end portions (22) are fixedly attached (e.g. via adhesive, bonding, or stitching) to the first lateral side region (13) of the sleeve. The fastening bands (2) are attached (here fixedly attached, and in alternative embodiments releasably attached) onto the sleeve such each band extends in substantially the transverse direction of the sleeve, with its distal end portion (24) positioned away from the central portion (14) of the sleeve. It can be seen in the exemplary embodiment that proximal end portions of the fastening bands may be bridged and/or integral to one another for ease in assembling the set of fastening bands onto the sleeve and/or for increasing the security of attachment of the fastening bands to the sleeve. It can be appreciated that each band in its extended configuration has a first major surface (34, not numbered on Figure 1) facing inwardly and a second major surface (33) facing outwardly and since in this exemplary embodiment the proximal end portions of the bands are attached to the outer surface (3) of the first lateral side region (13), the first major surface across the width of the fastening band is located towards the outer surface of the sleeve and a second major surface is located away from the outer surface of the sleeve. The second major surface (33) at the distal end portion (24) of the band favorably comprises mechanical (e.g. male) fastening elements (25). In the illustrated exemplary embodiment, the second major surface at the proximal end portion (22) of the fastening bands has a structure or is provided with a structure (26) that is adapted to be engaged by said mechanical (e.g. male) fastening elements (e.g. a loop engagement material). The second major surface at the inner band portion of the fastening band may also have a structure or is provided with a structure (26) that is adapted to be engaged by said mechanical (e.g. male) fastening elements on the second major (outer) surface (33) at the distal end portion (24).

In alternative embodiments, the outer surface of at least the first lateral side portion of the sleeve may in addition or alternatively have a structure or be provided with a structure that is adapted to be engaged by the mechanical fastening elements on the second major surface of the distal end portion of the fastening bands. For those embodiments in which the outer surface of at least the first lateral side portion of the sleeve comprises an engaging structure rather than the proximal end portion of the fastening bands, the proximal end portion can be configured much smaller than that shown in exemplary embodiment of Figure 1. This can be appreciated from the exemplary embodiment depicted in Figure 13. In this embodiment like the embodiment of Figure 1, the second major surface (33) at the distal end portion (24) of the fastening bands (2) favorably comprises mechanical fastening elements (25), in particular male fastening elements (e.g. hook-shaped or mushroom shaped fasteners), while now the outer surface (3) of the first lateral side region (13) is provided with a structure (29) that is adapted to be engaged by said mechanical (e.g. male) fastening elements (e.g. a loop engagement material). See also Figure 14. It will be appreciated that in alternative embodiments, instead of having the two lateral side regions each being provided with (e.g. laminated to) e.g. a loop engagement material, the entire outer surface of the sleeve may be provided with such a material. Returning to the exemplary embodiment depicted in Figure 13, it can be seen that the proximal end portions (22) of the fastening bands (2) do not include an engaging structure and are much smaller in size in the transverse direction, being configured to sufficiently allow for the attachment (typically fixed attachment) of the fastening bands to outermost portion of the first lateral side region (13) along and adjacent to the first lateral side edge (9). It will be appreciated that in such embodiments the proximal end portions of the fastening bands could alternatively be attached onto the inner surface of the sleeve, again along and adjacent to the first lateral side edge.

In general, fastening bands are favorably configured such that the second major surface at the distal end portion of the fastening bands is provided with male fastening elements, in particular male fastening elements selected from the group consisting of hook fasteners, mushroom-shaped fasteners, stem-shaped fasteners, cup-shaped fasteners, T-shaped fasteners, pin-shaped fasteners and mixtures thereof (referred to in the following as first band fasteners). When the proximal end portions of the fastening bands are attached to the outer surface of the sleeve, the second major surface at the proximal end portion of the fastening bands may have a structure or may be provided with a structure that is adapted to be engaged by said first band fasteners. In addition or alternatively, the outer surface of at least the first lateral side region may have a structure or may be provided with a structure that is adapted to be engaged by said first band fasteners. The second major surface at the inner band portion of the fastening bands may also have a structure or may be provided with a structure that is adapted to be engaged by the first band fasteners. It will be appreciated that a particular region or portion may be provided with the relevant engagement structure by laminating an appropriate layer of material onto the relevant region or portion.

For those embodiments in which the fastening bands are releasably attached to the outer surface of the sleeve, favorably the first major surface at the proximal end portions of the fastening bands is provided with male fastening elements, in particular male fastening elements selected from the group consisting of hook fasteners, mushroom-shaped fasteners, stem-shaped fasteners, cup-shaped fasteners, T-shaped fasteners, pin-shaped fasteners and mixtures thereof (referred to in the following as second band fasteners), while the outer surface at the first lateral side region of the sleeve has a structure or is provided with a structure that is adapted to be engaged by said second band fasteners. Here the first major surface at the proximal end portion of the fastening bands can then be releasably attached to the outer surface at the first lateral side region of sleeve. The second band fasteners may be identical to the first band fasteners or different. Favorably, the second and first band fasteners are different, where then the outer surface at the first lateral side region of the sleeve has a structure or is provided with a structure that is adapted to be engaged by both the first and second fasteners. More favorably, the second band fasteners and the relevant complementary engagement structure are selected such that they provide a stronger fastening (e.g. higher peel strength) than the first band fasteners and the relevant complementary engagement structure.

The exemplary embodiment depicted in Figure 15 is an example of a compression system (100) where the fastening bands (2) are releasably attached to the outer surface (3) of the sleeve (1). This exemplary compression system is similar the exemplary embodiment depicted in Figure 1, where the secondary closure element (5) is the same and the sleeve (1) and fastening bands differ to e.g. allow releasable attachment of the latter to the former. In particular in this exemplary embodiment, the first major (inner) surface (34) at the proximal end portion (22) of the fastening bands (2) is provided with mechanical fastening elements (in particular male fastening elements) (27) and the entire outer surface (3) of the sleeve (1) is provided e.g. via lamination of an appropriate material (e.g. a loop engagement material), with a structure (38) that is adapted to be engaged with said mechanical (male) fastening elements (27). This is best seen in Figure 16, showing a cross-sectional view of the sleeve in Figure 15, showing how the inner surface (34) at the proximal end portion (22) of the fastening band is releasably attached to the outer surface (3) at first lateral side region (15) of the sleeve (1). It will be appreciated that favorably the engaging structure (38) provided on the outer surface of the sleeve is adapted to be engaged with the mechanical (male) fastening elements (61) provided on the exterior flap (51) of the secondary closure element (5). In this exemplary embodiment, both the first and second lateral side regions (13, 15) of the sleeve are desirably configured such that they are trimmable, and as mentioned above such a configuration allows for an advantageously high degree of freedom in regard to width and/or circumference adjustment especially for those users having an unusual limb width or circumference where a custom sizing normally would be required.

Regardless of whether the fastening bands are fixedly attached or releasably attached to the first lateral side region of the sleeve, it may be advantageous (e.g. for ease in production or assembly purposes) in that the first lateral side region, the central region and second lateral side region are configured the same, i.e. the outer surface at each region has a structure or is provided with a structure that is adapted to be engaged by at least the mechanical fastening elements on the inner surface of the exterior flap, in particular by both the mechanical fastening elements on the inner surface of the exterior flap(s) and on the second major surface at the distal end portion of the fastening bands, and in the event of releasably attached fastening bands, then more particularly by all three types of the mechanical fastening elements, i.e. those on the inner surface of the exterior flap(s), those on the second major surface at the distal end portion of the fastening bands and those on the first major surface at the proximal end portion of the fastening bands. For embodiments with fixedly attached fastening bands, the mechanical fastening elements on the inner surface of the exterior flap(s) and on the second major surface at the distal end portion of the fastening bands may be identical. For embodiments with releasably attached fastening bands, as mentioned above mechanical fastening elements on the second major surface at the distal end portion of the fastening bands and on the first major surface at the proximal end portion of the fastening bands are favorably different, where the latter ones (i.e. second band fasteners) and the relevant engagement structure provided a stronger fastening (e.g. higher peel strength) that the former ones (i.e. the first band fasteners) with same engagement structure. For such embodiments the mechanical fastening elements on the inner surface of the exterior flap(s) may be identical to either the second band fasteners or the first band fasteners.

The distal end portions and/or inner band portions of fastening bands desirably have a height relative to the transverse direction of the sleeve of at least 1 cm, more desirably as least 2 cm. most desirably at least 3 cm. The distal end portions and/or inner band portions of fastening bands desirably have a height relative to the transverse direction of the sleeve of at most 10 cm, more desirably at most 8 cm, most desirably at most 6 cm. The proximal end portions typically have at least the same height as the distal end portions and/or inner band portions of fastening bands. As mentioned above, favorably the proximal end portions of fastening bands are bridged and/or integral to one another. The portion of the fastening bands extending beyond the first lateral side edge desirably has a width relative to the transverse direction of the sleeve of at least 3 cm, more desirably at least 6 cm. The portion of the fastening bands extending beyond the first lateral side edge desirably has a width relative to the transverse direction of the sleeve is at most 25 cm.

Returning to the exemplary embodiment depicted in Figure 1, the secondary closure element (5), which is favorably configured as a tongue, is two-fold releasably attachable to the sleeve (1), i.e. to the fastening bands (2) provided in the first lateral side region (13) of the sleeve and to the second lateral side region (15) of the sleeve, in particular along the second lateral side edge (10). In particular it can be seen in Figure 1, that the secondary closure element (5) of the compression system is favorably provided with a plurality of rings (16) in series lengthwise between the upper and lower edges (77, 88) of the closure element. There is a single ring provided for each of the fastening bands, such that when the closure element is (releasably) attached to the sleeve e.g. along the second lateral side edge (10), there is a ring located opposite to each of the fastening bands. In particular, the fastening bands (2) and rings (16) are configured and arranged such that, in use the bands can be passed through the rings then turned back on themselves, such that the first lateral side edge (9) of the sleeve (1) is drawn towards the rings and thus sleeve is tightened about the limb of the use , and then the fastening bands fastened so that the sleeve is restrained about the limb of the user, more particularly the first lateral edge is drawn towards the second lateral edge (10) of the sleeve, but the two lateral edges of the sleeve do not overlap.

In addition the closure element (5) is provided with a plurality of pairs of flaps (50) extending lengthwise in series to one side of the series of rings (16). As indicated above, in alternative embodiments, the closure element may be provided with a single elongate pair of flaps extending lengthwise to one side of the series of rings; such an exemplary embodiment is illustrated in Figure 13 wherein the secondary closure element (5) is provided a single elongate pair of flaps (50) extending lengthwise to one side of the series of rings (16) rather than a series of such pairs. Each pair of flaps includes an exterior flap (51) overlying an interior flap (52). As can be seen in the exemplary embodiment of Figure 1, the interior flap of each of the pair of flaps is bridged and integral to the interior flap(s) of the neighboring pair(s) so that the interior flaps are provided in a form from a single elongate strip. It will be appreciated that the interior flaps can be alternatively provided in the form of a series of individual, separate (shorter) strips, like the exterior flaps. As can be appreciated from the embodiment shown Figure 1, in particular from the cross-sectional view in Figure 2, the opposing inner surfaces of the exterior and interior flaps (51, 52) comprise mechanical fastening elements (61, 62); said mechanical fastening elements (61, 62) being adapted to releasably engage the outer and inner surfaces (3, 4) respectively, of at least the second lateral side region (15) of the sleeve (1). It will be appreciated that each of the inner and outer surfaces of at least the second lateral side region of the sleeve may have a structure (e.g. integral to the particular material used for the sleeve) or may be provided with a structure (e.g. lamination of a second engaging material on the underlying material used for the sleeve) that is adapted to be engaged by mechanical fastening elements provided on inner surface of the interior and exterior flaps, respectively. Furthermore for each pair of flaps, at least the exterior flap (51) is movable relative to the interior flap (52) such that the exterior flap is capable of performing a hinge movement about a longitudinal or substantially longitudinal axis (L) and relative to the interior flap. This can be best seen in Figure 1 to the upper left; here one of the exterior flaps is shown in an open position where in a hinged movement along the axis (L) the exterior flap is moved away from the interior flap and thus exposing its inner surface and mechanical fastening elements. The axis (L) for hinged movement of the exterior flap(s) (51) is positioned towards (proximal) to the series of rings (16). It can be seen that favorably the series of rings (16) are configured and arranged such that in use in when the closure element (5) is attached to the sleeve along the second lateral side edge (10) of the sleeve (1), the series of rings will be located distal the second lateral side edge.

As will be become evident in the description below about how to use compression systems described herein, the secondary closure element is configured and arranged relative to the sleeve such that, in use when the closure element is attached to the sleeve along the second lateral side edge and the fastening bands fastened, the closure element is generally centrally positioned adjacent to and extends along the first and second lateral side edges of the sleeve, so that the closure element is located between the user and an opening defined between the first and second lateral edges of the sleeve (for example see Figure 11 which show the compression system as it would look wrapped and tightened about the limb of a wearer).

Referring to the cross-sectional view shown in Figure 2, it can be recognized that each exterior flap (51) is affixed (in particular indirectly affixed) to the outer surface of the closure element (5) along the axis for hinged movement. It can also be recognized that the elongate strip forming the interior flaps is also affixed (in particular directly affixed) to the outer surface of the closure along the said axis for hinged movement. In alternative embodiment, the interior flap(s) may be completely affixed onto the outer surface of the closure element.

Generally, the mechanical fastening elements provided on the inner surfaces of the flaps favorably comprise male fastening elements, more favorably male fastening elements selected from the group consisting of hook fasteners, mushroom-shaped fasteners, stem-shaped fasteners, cup-shaped fasteners, T-shaped fasteners, pin-shaped fasteners and mixtures thereof (referred to in the following as flap fasteners or more specifically interior and exterior flap fasteners). The interior band fasteners may be identical to the exterior band fasteners or different. Similarly the structure of or provided on the inner surface of the sleeve may be identical to the structure of or provided on the outer surface of the sleeve.

As mentioned above, it has been found desirable to configure and arrange the inner surface of the second lateral side region of the sleeve and the mechanical fastening elements of the inner surface of the interior flap so as to provide shear strength of at least 2 N/cm² as measured according to EN13780. In addition or alternatively, it has been found desirable to configure and arrange the inner surface of the second lateral side region of the sleeve and the mechanical fastening elements of the inner surface of the interior flap so as to provide peel strength less than 0.6 N/cm, more favorably equal to or less than 0.3 N/cm, as measured according to EN 12242.

Also as mentioned above it has been found desirable to configure and arrange the outer surface of the second lateral side region of the sleeve and the mechanical fastening elements of the inner surface of the exterior flap so as to provide shear strength of at least 7 N/cm² as measured according to EN13780. In addition or alternatively, it has been found desirable to configure and arrange the inner surface of the second lateral side region of the sleeve and the mechanical fastening elements of the inner surface of the interior flap so as to provide peel strength so as to provide a peel strength of at least 0.6 N/cm as measured according to EN 12242. In addition or alternatively, it has been found desirable to configure and arrange the inner surface of the second lateral side region of the sleeve and the mechanical fastening elements of the inner surface of the interior flap so as to provide peel strength so as to provide a peel strength of at most 10 N/cm, more desirably at most 5 N/cm, as measured according to EN 12242.

Examples of mechanical fastening elements tapes that may be suitable for use on the inner surface of the interior flap include low profile, extruded flexible film fastener tapes having mushrooms as engaging elements, such as those marketed by Gottlieb Binder GmbH & Co KG under the trade designation MICROPLAST, e.g. item nos. 25442, 25443, 25445, 25446 and 27443 or those marketed by 3M Deutschland GmbH (health care business) under the product number #7334. Examples of mechanical fastening elements tapes that may be suitable for use on the inner surface of the exterior flap include woven mushroom tapes marketed by Velcro USA Inc. under the trade designation VELCRO (e.g. hook 088) or VEL-LOC or SUPER VEL-LOC (e.g. items 085, 083 or quadrilobal) or extruded hook tapes marketed by Alfatex Ltd. under the trade designation GRIPPER (e.g. medium pp). For example, when using the material used in the aforesaid wrist splint/brace product marketed under the trade designation 3M FUTURO or in the aforesaid compression legging marketed under the trade designation JUXTA-CURES as sleeve material for a compression system as described herein with the smooth side as the inner side of the sleeve and the opposite wooly/fuzzy side as the outer side of sleeve, e.g. the MICROPLAST product (in particular Item No. 25443) is useful for providing the mechanical fastening elements for the interior flap, while the GRIPPER product (in particular medium PP type thereof) is useful for providing the mechanical fastening elements for the exterior flap. In these examples the sleeve material is used as is. Alternatively the material used for the sleeve may be laminated to a loop engagement material. For example, an unbroken loop (UBL) knit Nylon™/Spandex™ fabric marketed under the product number WW1306 by Gehring-Tricot Corp., Garden City, NY 11530, USA, may be laminated to one side of the polyester and Spandex™ based spacer fabric marketed under the trade designation SHR 755 D3 by Gehring-Tricot Corp, the loop engagement material side forming the outer side of the sleeve. For such a sleeve laminate, the 7334 fastener product of 3M Deutschland GmbH is useful for providing the mechanical fastening elements for the interior flap, while the aforesaid fastener products marketed by Velcro Inc. are useful for providing the mechanical fastening elements for the exterior flap.

Secondary closure elements, in particular secondary closure elements configured in the form of a tongue, may favorably include one or more elongate stiffeners extending lengthwise. As mentioned above, stiffeners may be made of e.g. metal or thermoplastic materials including thermoformable thermoplastic materials (such as polypropylene, polyamide, polyester (e.g. 3M Scotchcast Thermoplastic Material 72362)). For stiffeners having a width greater than five millimeters, it may be favorable to provide them with perforations to allow for breathability. For design and/or fixing purposes, stiffeners may be positioned on the surface of the appropriate part(s) of the closure element, which are then covered completely with a sheet of fabric that is sewn or laminated onto the respective part(s) of the closure element.

Secondary closure elements may comprise a spacer fabric and/or a foam material. Moreover the closure element comprises a multilayer construction comprising at least one layer, in particular two or more layers, of a material selected from the group consisting of a spacer fabrics, foams or combinations thereof. Suitable foams include memory foams, in particular high density memory foam. High density memory foams are memory foams that have a density of at least 65 kg/m3, in particular at least 70 kg/m³, more particularly at least 85 kg/m³, most particularly at least 105 kg/m³. Examples of suitable memory foams, include high density memory foams available from Filtrona Porous Technologies GmbH marketed under the trade designations SRF EP2, Argus, Argus Soft, and Argus Supersoft. Examples of suitable spacer fabrics include polyester spacer fabrics available from Muller Textil GmbH, 51674 Wiehl-Drabenderhöhe, Germanymarketed under the product numbers 5754 and 6018. Closure elements, excluding structural elements attached thereto (e.g. flaps, rings, etc.), favorably have a thickness from 2 mm to 12 mm, inclusive, in particular a thickness from 3 mm to 8 mm, inclusive. In a region free of seams, stiffeners, if applicable, and attached structural elements (e.g. flaps, rings, etc), closure elements favorably have an air permeability equal to or greater than 40 cm/s, more favorably equal to or greater than 80 cm/s, even more favorably equal to or greater than 120 cm/s, most favorably equal to or greater than 160 cm/s, as measured according to ISO 9237:1995 using a test pressure of 200Pa and/or a water vapor transmission rate equal to or greater than 1000 g/ (m² ×24 h), more favorably equal to or greater than 1500 g/ (m² ×24 h), even more favorably equal to or greater than 2000 g/ (m² ×24 h), most favorably equal to or greater than 2500 g/ (m² ×24 h), as measured according to ISO 15106, part 1.

Returning to the exemplary embodiment depicted in Figure 1 and the cross-sectional view of the closure element in Figure 2, it can be seen, that the closure element (5) includes an elongate stiffener (42) extending lengthwise. Desirably the elongate stiffener is positioned relative to the rings such that in use when the sleeve is tightened, the rings are located towards the one side the stiffener, in particular the elongate stiffener is positioned relative to the ring and flaps such that in use when the sleeve is tightened, the stiffener is essentially located between the rings and flaps. The stiffener is located between two layers, an inner layer (6) may of a resilient material (e.g. a spacer or foam material) and an upper layer (36) made of an appropriate covering material.

In the exemplary embodiment of Figure 1, each ring (16) is connected to the closure element (5), in particular to the outer surface of the closure element, by a strap (17). From the cross-sectional view in Figure 2, it can be seen that one end of the strap bears the ring and the other end is attached (either directly or indirectly; favorably fixedly), to the closure element, in particular to the outer surface thereof, such that the strap extends between the closure element and the ring in substantially the transverse direction relative to the sleeve. The end of strap which is attached to the closure element is desirably located proximal to the pairs of flaps, in particular proximal to the axis of hinged movement of the exterior flap, while the end of the strap which bears the ring is located distal to the pair of flaps.

Desirably straps comprise an expandable strap portion comprising a material having elasticity in at least the transverse direction (relative to the sleeve) and being configured and arranged such that when the expandable strap portion is in its non-expanded state (e.g. when the compression system is not in use) there is exteriorly a loop of material rising outwardly and when, in use when the closure element is attached to the sleeve and tension is provided in the transverse direction of the sleeve, the expandable strap portion expands in the transverse direction and the loop flattens (eventually disappearing).

Returning to the exemplary embodiment depicted in Figure 1, in particular to the cross-sectional view of Figure 2, it can be seen that each strap (17) include an expandable portion (21) that is configured with a loop (20) towards the exterior and rising outwardly. In particular, it can be seen that from the top, the indicating loop has the general form of a mound or a hump, while the loop-like form can be best seen in cross-sectional view shown in Figure 2 which like Figure 1 shows the expandable strap portion in its non-expanded state.

It can also be seen that the expandable portion of the strap favorably comprises two layers, an outer layer of material (18) and an inner layer of material (19) and wherein the inner layer of material is affixed to the outer layer of material, so as to provide a loop of outer-layer-material (i.e. loop (20)) above the inner layer when the expandable strap portion is in its non-expanded state, and which in use under the provision of tension and accordingly expansion of expandable strap portion in the transverse direction, respectively, can flatten.

For those embodiments where the expandable portion of the strap includes two layers, favorably the product of the modulus of elasticity (in the transverse direction) of the inner-layer-material times the thickness of the inner-layer material is less than the product of the modulus of elasticity (in the transverse direction) of the outer-layer-material times the thickness of the outer-layer material. More favorably, the product of the modulus of elasticity of the inner-layer-material times the thickness of the inner-layer-material is at least a factor of two times, more favorably at least a factor of four times, lower the product of the modulus of elasticity of the outer-layer-material times the thickness of the outer-layer-material, so that the transition from a pronounced loop to a completely flattened out loop does not require too much elongation in terms of length. Favorably at least one of the outer-layer- and inner-layer-materials has elasticity in at least the transverse direction, more favorably inner-layer-material has elasticity in at least the transverse direction.

Modulus of elasticity (also called elastic modulus) may be determined in accordance to ASTM D 882-09 entitled "Standard Test Method for Tensile Procedure of Thin Plastic Sheeting". It is to be noted that although the standard expressly states that it covers the determination of tensile properties of plastics in the form of thin sheeting, it has been found that the described test method and determination of modulus of elasticity may be suitably used in regard to materials suitable for use in compression devices described herein.

It will be appreciated from Figure 2 that for easing in assembling and production, when the outer-layer material of the strap has a stretch less than 10% down to zero under a load of 10 N per cm width, it may be favorable to provide the outer layers of the strap (17) and the outer layer of the exterior flap (51) as single integral component, e.g. in a single elongate strip of the particular material. In regard to the strap, it will be appreciated that the loop-containing portion (21) of the strap is expandable in at least the transverse direction is a result of inner and/or outer-layer material properties, the configuration of the attachment of the inner and outer-layer-materials to one another as well as the configuration of the attachment of the strap to the ring and onto the closure element. When the expandable portion of the strap is not expanded, i.e. in its non-expanded state, as shown in Figures 1 and 2, the loop (20) is visible as an elongate mound or hump. In use, when the system is applied onto the limb of a user and tension is applied onto the system, in particular onto sleeve in the transverse direction, the expandable portion (21) of the strap will accordingly expand in the transverse direction and the loop can and will flatten.

For those favored embodiments where the sleeve comprises a short-stretch material as described above, to further underline the indicating effect of the loop-indicating configuration and/or to further facilitate the provision of uniformity of compression, desirably the product of the modulus of elasticity (in the transverse direction) of the material of the loop (e.g. the outer-layer-material in the two-layer expandable strap portion) times the thickness of the material of the loop is favorably at least 90% of the product of the modulus of elasticity (in the transverse direction) of the short-stretch material times the thickness of the short-stretch material, in particular the product of the modulus of elasticity of the material of the loop (e.g. the outer-layer-material in the two-layer expandable strap portion) times the thickness of the material of the loop is equal to or greater than the product of the modulus of elasticity of said short-stretch material times the thickness of the short-stretch material.

For ease in viewing the loop-indicating configuration, the expandable portion of the strap favorably has in its non-expanded state a width relative to the transverse direction of the sleeve of at least 0.1 cm, in particular at least 0.5 cm. To facilitate favorably system construction e.g. in terms of sizing of components and material use, desirably the expandable portion of the strap has in its non-expanded state a width relative to the transverse direction of the sleeve of at most 4 cm, in particular at most 3 cm. Favorably, especially in regard to facilitating viewing of changes between a pronounced loop-configuration and flattened configuration, the expandable portion of the strap has in its expanded state at the point where the loop just fully flattens out a width relative to the transverse direction of the sleeve of at least 1 cm. For ease in viewing and/or to facilitate uniformity of compression in the transverse/ circumferential direction, the expandable portion of the strap favorably has in its expanded state at the point where the loop just fully flattens out a width relative to the transverse direction of the sleeve is at most 8 cm, in particular at most 6 cm. Generally, for compression systems including rings and straps having an expandable portion with a loop-indicating configuration as described above, favorably each strap has a height relative to the transverse direction of the sleeve of at least 1 cm, more favorably at least 2 cm, and most favorably at least 3 cm. Favorably each strap has a height relative to the transverse direction of the sleeve of at most 10 cm, more favorably at most 8 cm, and most favorably at most 6 cm.

To further facilitate the provision of a favorable anatomic fit over the portion of the limb (e.g. the lower leg including the calf) of a user that is covered by the sleeve of the system in use, favorably the plurality of rings or fastening bands including the interstices between rings or bands, respectively extends over a height corresponding to at least 70% of the height of the sleeve from the upper to lower edge. Generally it is desirable that the plurality of rings or bands including the interstices between rings or bands, respectively extends a height corresponding from 70% up to and including 100%, and possibly greater than 100% of the height of the sleeve from the upper to lower edge. The height of the interstices between rings or bands may range from 0.1 mm to 7 cm, inclusive, in particular from 0.3 mm to 3 cm, inclusive, and more particularly from 0.5 mm to 2 cm.

To facilitate application and an overall smooth fit of the device, rings are favorably configured and/or selected, such that the opening of the ring has a height relative to the transverse direction of the sleeve which is greater than the height relative to the transverse direction of the sleeve of the fastening band. And for those embodiments including rings and straps, favorably the rings are configured and/or selected, such that the opening of the ring has a height relative to the transverse direction of the sleeve which is greater than the height relative to the transverse direction of the sleeve of the strap. In addition or alternatively, rings may be made of metallic or polymeric material or may be created or provided in a web material (e.g. in the form of an eyelet). In addition or alternatively, rings are desirably rectangular or substantially rectangular in form; or oval or substantially oval in form (e.g. narrow or elongate oval, canoe-form, elongate teardrop); or an elongate or narrow D-shape in form.

The exemplary embodiment depicted in Figures 17 and 18 is an example of a compression system (100) where the fastening bands (2) are fastened directly onto appropriate complementary engaging structure (55) provided on the secondary closure element (5). In addition, the fastening bands are integral with the first lateral side region (15) of the sleeve (1). In regard to the latter, it can be seen in Figure 17 to the right that the fastening bands (2) extend in substantially the transverse direction of the sleeve out from the first lateral side edge (9) of the sleeve (1). As mentioned above, in such cases the first lateral side edge of the sleeve will be understood to run along a line (marked in Figure 17 with a dashed line and labeled E) including the outer edges of the first lateral side region (15) of the sleeve located between fastening bands (9-1) and, if applicable, next to the uppermost and/or lowermost fastening bands and the boundary (9-2) between the first lateral side region of the sleeve and the fastening band. Referring to Figure 18 to the right, it can be seen that the first major (inwardly facing) surface (44) at the distal end portion (24) of the fastening bands is provided with a mechanical (male) fastening elements (56). It will be appreciated that in comparison to the exemplary embodiments including rings, the extent of which the fastening bands extend over the first lateral side edge will normally be much less. Looking at the left side of Figures 17 and 18, it can be seen that in the exemplary embodiment the outer surface of the secondary closure element (5) is provided with a structure (55) that is adapted to be engaged by the mechanical fastening elements on the first major surface of the distal end portion of the fastening bands, e.g. a loop engagement material. In particular an individual patch of said engaging structure is provided for each of the fastening bands, wherein the pairs of flaps (50) extend lengthwise to one side of the series of engaging structure patches. Similarly to the other exemplary embodiments described above, the axis (L) for hinged movement of the exterior flaps (51) is positioned proximal to said engaging structure, and in use, when the closure element is attached to the sleeve along the second lateral side edge (10) of the sleeve, the engaging structure for the fastening bands is located distal to the second lateral side edge. It will be appreciated that in alternative embodiments, a single large section of engaging structure could be provided or the outer surface of the secondary closure element could have inherently have a structure that can engaged the fastening elements.

It will be appreciated that in each of the exemplary embodiments of compression depicted in Figures 1, 13, 15 and 17, the flaps comprise web laminates, whereby the relevant portion of the inner surface of the outer web material forming the flap is provided with mechanical fastening elements via lamination of an appropriate web- or tape-like fastener material onto outer web material. In alternative embodiments, the pair of flaps may be favorably injection molded components. Such an exemplary embodiment is shown in Figure 19.

The exemplary embodiment depicted in Figure 19 is an example of a compression system where each of the pairs of flaps is an injection molded assembly. This exemplary compression system (100) is similar to the exemplary embodiment depicted in Figure 1 with some exceptions. The outer forms of the closure element (5) and the set of fastening bands (2) on the sleeve (1) are somewhat different, and each of the pairs of flaps (50) is provided as a part of an injection molded assembly. The assemblies are attached in series lengthwise to the outer surface of the closure element, all to one side of the rings (16) and near to the end of the ring-bearing strap (17) which also attached to the outer surface of the closure element. Similar to the other embodiments, for each pair of flaps, at least the exterior flap (51) is movable relative to the interior flap (52) such that the exterior flap is capable of performing a hinge movement about a longitudinal or substantially longitudinal axis (L) and relative to the interior flap. This can be best seen in Figure 19 to the upper left where the exterior flap (51) of the uppermost pair of flaps is in an open position; the exterior flap being moved away from the interior flap (52) in a hinged movement along the axis (L). In addition, each flap assembly (50) includes a locking element (53). This can be best recognized in the Figure 19 to the left, in the particular in the pair of flaps second from the top, where the locking element (53) can be seen its unlocked position towards the ring and where the exterior flap is in a closed position. In the remaining four pairs of flaps, the exterior flaps are in closed positions and the locking elements are locked positions. Also here the indicating loops (20) of the straps (17) can be recognized. It can be seen that the expandable portion (21) of each of the straps, in particular the outer-layer includes an opening (40). The opening can be recognized as a semi-circle to the right side of the indicating loop (20). The upper surface of the inner layer of the expandable portion can include colors or other indicia, that becoming visible when the expandable portion is expanded.

The operation of the flap assembly (50) can be best understood in Figures 20 and 21 showing top and central cross-sectional views, respectively, of one flap assembly (50) from left to right in locked, unlocked but closed, hinged open, closed but unlocked, and locked configurations. To unlock the assembly, the locking element (53) is turned counter-clockwise (see arrow in outermost left figure) 180° along a pivot axis (X) (compare the two outer left figures). After unlocking, the exterior flap (51) can be opened in a hinged movement along the longitudinal axis (L) thereby uncovering the interior flap (52) and exposing the mechanical fastening elements of both the interior and exterior flaps (62, 61, respectively) (see the central figure). After closing the exterior flap (51) (see next figure to the right), the locking element (53) can be locked by turning clockwise (see arrow) 180° along the pivot axis X (compare the two outer right figures).

Although not specifically shown in the exemplary embodiments depicted herein, compression systems described herein may be configured to include other structural elements, for example a foot portion extending from the sleeve, in particular extending from an appropriate portion of the lower edge of the sleeve. Such a foot portion may be configured and arranged in the form of a stir-up or alternatively such as foot portion may be configured to provide a more extensive covering of the foot. Moreover the sleeve and such a foot portion may be configured and arranged so as to provide a boot-like compression device, either closed or opened toed and/or either closed or opened heeled. Such a foot part may be provided integrally with the sleeve or alternatively as a separate component that can be attached to the sleeve by an appropriate fastening means, such as buttons, mechanical fasteners and the like. Compression systems may also include bladders or gel inserts to facilitate modification of circumferential size. In this regard, sleeves, for example, could be provided with double walls or interior pockets for such inserts so that such insert(s) may be inserted and/or removed as needed or desired. If desired, compression systems described herein can be used in conjunction with stockings.

Figures 3 to 12 provide a series of illustrations of an exemplary way of applying a compression system depicted in Figure 1.

Referring to Figures 3 and 4, in a first couple of steps (which can be done in any order), each of the exterior flaps (51) is moved in a hinge-movement away from the underlying interior flap (52) thereby exposing its the inner surface and mechanical fastening elements (62) thereon, and the fastening bands (2) are be fastened in conjunction with secondary closure element (5), (e.g. using the opposing rings (16) on the closure element) thereby attaching the secondary closure element to along the first lateral side edge (9) of the sleeve (1) via the bands. Desirably the secondary closure element is preliminarily attached to the sleeve via the bands at a position which will allow for tightening in a later step. As can be recognized in the cross-sectional view provided in Figure 4, the expandable strap (17) is in a non-expanded state whereby there is a loop of material (20) rising outwardly and exteriorly.

Referring to Figure 5, in the next steps, the secondary closure element (5) of the compression system (100) is positioned along the limb (not shown) of the user to be treated and the sleeve (1) is wrapped around the limb moving generally from the attached side of the sleeve to the unattached side, where a part of the sleeve (e.g. a part of the second lateral side region (15)) is placed over the inner surface of the interior flap(s) (52; not visible, position marked with a dashed line) on the secondary closure element so that sleeve covers the limb in a selected manner and fit and such that the inner surface of the sleeve releasably engages with the mechanical fastening elements provided on the inner surface of the interior flap(s).

For compression systems suitable for use with the lower leg of the user favorably the system is configured and arranged such that in use the secondary closure element will be positioned in the front and the central region of the sleeve will typically be positioned at least around the back and thus accordingly next to the calf muscles. For those embodiments including rings or fastening-bands-engaging-structure on the secondary closure element, favorably the system is configured and arranged such that in use the rings or said fastening-band-engaging structure on the secondary closure element will generally be positioned towards the front, in particular so that they extend generally either along the tibia or near the length of the tibia.

As illustrated in Figure 6, in the event there is any excess and unengaged material of the sleeve (1) extending beyond the inner surface (as typically expected), in particular beyond the mechanical fastening elements of the interior flap(s) distal to the central portion of the sleeve, this is trimmed off e.g. with a pair of scissors (99). After such trimming, a configuration of the compression system (100) such as that shown in Figure 7 will be obtained. Alternatively if the sleeve is by chance just the correct size, the configuration as shown in Figure 7 may be obtained directly upon attachment of the inner surface of the sleeve to the interior flap(s) in the previous step. In either case, referring to Figure 8 showing a cross-sectional view of part of the system, the inner surface (4) of that portion of the sleeve (1) that is adjacent to the second lateral side edge (10) (either newly created upon trimming or the original one) is engaged with the mechanical fastening elements (62) of the interior flap(s) (52), while at this point the exterior flaps (51) are still hinged away the interior flap(s), the mechanical fastening elements (61) of the exterior flaps being thus un-engaged.

Referring to Figures 9 and 10, in a next step, the exterior flaps (51) are placed into contact with the outer surface (3) of the sleeve (1), e.g. via a hinged movement along the longitudinal axis L toward the interior flap(s) (52), so that the mechanical fastening elements (61) provided on the inner surface of the exterior flaps releasably engage the outer surface of the sleeve, in particular the outer surface of the portion of the sleeve whose inner surface (4) is releasably engaged with the fastening elements (62) of the interior flap (52). As can be appreciated from Figures 9 and 10, the outwardly facing loop (20) on the ring-straps (17) are still observably, because although the sleeve of the compression system (100) is fastened along both the lateral ends (9,10), the compression system is not yet tightened to provide a desired and/or needed level of compression.

Once the system has been applied about the limb and properly sized as described above, favorably the method includes the steps, fastening the fastening bands; repositioning to the fastening bands so as to tighten the sleeve about the limb of the user and provide a selected level of compression; and re-fastening the fastening bands.

Referring to Figures 11 and 12, each of fastening bands (2) can be un-fastened (e.g. while keeping the fastening bands threaded through its opposing ring (16), turned back on itself) and then the fastening band can pulled such that the first lateral side edge (9) of the sleeve (1) is drawn towards the rings (16), in particular towards the second lateral side edge (10), so that the sleeve (1) is tightened about the limb of the user, wherein each fastening band is pulled until the loop in the expandable portion (21) of the opposing ring strap (17) fully flattens out. Once the loop disappears i.e. is no longer visible because it flattened out, the fastening band is fastened. Once all the fastening bands are so fastened, the compression system (100) is correspondingly restrained and tightened about the limb of the user to provide compression. Desirably the fastening of each band is performed when the loop of material has just fully flattened out (and accordingly the loop has just disappeared). When during use, the expandable portion of the strap starts to re-form its loop, due the tension decreasing, this will provide an indication to the user or care-giver that system needs to be re-tightened and/or re-applied.

Although Figure 11 does not shown the limb, it can be appreciated that when the exemplary compression system (100) is in use on the limb of the user, the sleeve will be disposed about a central axis and the plurality of rings extends will be located along a first axis, whereby relative to a projection of the first axis onto said plane containing the central axis, the first axis is in parallel or essentially parallel alignment relative to the central axis. Generally it is favorable that the first axis is either in parallel alignment relative to the central axis or nearly parallel alignment relative to the central axis (i.e. the first axis may be inclined forming an acute angle of no more than 5° relative to the central axis). It is to be appreciated that when the exemplary compression system is in use on the limb of the user, it is possible that the series of rings may not extend along a perfectly straight axis, i.e. its projection may be curved due to tension and particular leg geometry of the user, and in such cases the relevant axis along which the series of rings extends may be defined as being the axis resulting from a best linear fit (linear regression) to the projected curve. Further as it can be appreciated from the Figure 11, the secondary closure element (5) is favorably configured as a tongue, such that, in use, the closure element is generally centrally positioned adjacent to and extends along the first and second lateral edges (9, 10) of the sleeve (1), so that the closure element is located between the user and an opening defined between the first and second lateral edges of the sleeve.

## Claims

1. A compression system (100) for applying compression to a limb of a user comprising a sleeve (1) for substantially covering a portion of the limb of a user,
wherein the sleeve has an outer surface (3), an inner surface (4), an upper edge (7), a lower edge (8) and two lateral side edges (9, 10), wherein in the transverse direction from the first lateral side edge to the second lateral side edge the sleeve comprises a first lateral side region (13), a central region (14) and a second lateral side region (15),
the first lateral side region (13) of the sleeve being provided with a plurality of fastening bands (2) in series between the upper and lower edges of the sleeve and the second lateral side region (15) being trimmable,
and wherein the system further comprises a secondary closure element (5), said closure element being releasably attachable to the second lateral side region (15), said closure element further being provided with either an single elongate pair (50) of flaps extending lengthwise or a plurality of pairs (50) of flaps (51, 52) extending lengthwise in series,
said elongate pair of flaps or pairs of flaps, as applicable, each including an exterior flap (51) overlying an interior flap (52), wherein at least the exterior flap is movable relative to the interior flap such that the exterior flap is capable of performing a hinge movement about a longitudinal or substantially longitudinal axis (L) and relative to the interior flap and wherein the opposing inner surfaces of the interior and exterior flaps comprise mechanical fastening elements (61, 62) adapted to releasably engage the inner and outer surfaces (4, 3), respectively, of at least the second lateral side region (15) of the sleeve and to secure the secondary closure element along the second lateral side region of the sleeve in a clamp-like fashion.

2. A compression system according to claim 1, wherein each of the inner and outer surfaces (4, 3) of at least the second lateral side region (15) of the sleeve has a structure or is provided with a structure that is adapted to be engaged by mechanical fastening elements (61, 62) provided on inner surface of the interior and exterior flaps (51, 52), respectively.

3. A compression system according to any one of the preceding claims, wherein the mechanical fastening elements (61, 62) provided on the inner surfaces of the flaps (51, 52) comprise male fastening elements (25, 27) in particular male fastening elements selected from the group consisting of hook fasteners, mushroom-shaped fasteners, stem-shaped fasteners, cup-shaped fasteners, T-shaped fasteners, pin-shaped fasteners and mixtures thereof.

4. A compression system according to any one of the preceding claims, wherein the closure element (5) further comprises a locking element (53) or a series of locking elements (53), said locking element or element(s) being provided in association with the exterior flap (51) or exterior flaps (51), as applicable, said locking element(s) and exterior flap(s) being configured and arranged such that in use when the closure element (5) is attached to the sleeve (1), the locking element(s) (53) can be clamped onto the exterior flap(s) (51), in particular onto the outer surface of the exterior flap(s) (51).

5. A compression system according to any one of the preceding claims, wherein the closure element (5) includes one or more elongate stiffeners (42) extending lengthwise.

6. A compression system according to any one of the preceding claims, wherein the closure element (5) comprises a spacer fabric and/or a foam.

7. A compression system according to any one of the preceding claims, wherein each fastening band (2) comprises a distal end portion (24), each band (2) extending in substantially the transverse direction of the sleeve (1) with its distal end portion (24) positioned away from the central portion (14) of the sleeve.

8. A compression system according to claim 6, wherein an outer surface of the secondary closure element (5) has a structure or is provided with a structure (55) that is adapted to be engaged by mechanical fastening elements (56) on the first major surface (44) of the distal end portion (24) of the fastening bands (2) and wherein said pair (50) of flaps (51, 52) or pairs (50) of flaps (51, 52), as applicable, of the closure element (5) extend lengthwise to one side of said engaging structure (55) for the fastening bands (2).

9. A compression system according to any one of the claims 1 to 6, wherein the secondary closure element (5) is provided with a plurality of rings (16) in series such that when the closure element (5) is attached to the sleeve (1), each ring (16) is located opposite to a fastening band (2), and wherein said pair (50) of flaps (51, 52) or pairs (50) of flaps (51, 52), as applicable, extend lengthwise to one side of the series of rings (16).

## Patentansprüche

1. Kompressionssystem (100) zum Anwenden von Kompression auf eine Gliedmaße eines Nutzers, umfassend eine Hülle (1), um einen Abschnitt der Gliedmaße eines Nutzers im Wesentlichen abzudecken,
wobei die Hülle eine Außenoberfläche (3), eine Innenoberfläche (4), eine obere Kante (7), eine untere Kante (8) und zwei laterale Seitenkanten (9, 10) aufweist, wobei in der Querrichtung von der ersten lateralen Seitenkante zur zweiten lateralen Seitenkante die Hülle einen ersten lateralen Seitenbereich (13), einen zentralen Bereich (14) und einen zweiten lateralen Seitenbereich (15) umfasst,
wobei der erste laterale Seitenbereich (13) der Hülle mit einer Mehrzahl von Befestigungsbändern (2) in einer Reihe zwischen der oberen und unteren Kante der Hülle versehen ist und der zweite laterale Seitenbereich (15) zuschneidbar ist
und wobei das System ferner ein sekundäres Verschlusselement (5) umfasst, wobei das Verschlusselement lösbar an dem zweiten lateralen Seitenbereich (15) befestigbar ist, wobei das Verschlusselement ferner mit entweder einem einzigen länglichen Paar (50) Klappen, die in Längsrichtung verlaufen, oder einer Mehrzahl von Paaren (50) von Klappen (51, 52) versehen ist, die in einer Reihe in Längsrichtung verlaufen, wobei das längliche Paar aus Klappen oder die Paare aus Klappen, wie zutreffend, jeweils eine äußere Klappe (51) einschließen, die auf einer inneren Klappe (52) liegt, wobei mindestens die äußere Klappe relativ zur inneren Klappe derart beweglich ist, dass die äußere Klappe in der Lage ist, eine Scharnierbewegung um eine Längs- oder im Wesentlichen Längsachse (L) und relativ zur inneren Klappe durchzuführen und wobei die gegenüberliegenden inneren Oberflächen der inneren und der äußeren Klappen mechanische Befestigungselemente (61, 62) umfassen, die daran angepasst sind, die innere bzw. äußere Oberfläche (4, 3) von mindestens dem zweiten lateralen Seitenbereich (15) der Hülle lösbar zu ergreifen und das sekundäre Verschlusselement entlang dem zweiten lateralen Seitenbereich der Hülle klammerartig zu befestigen.

2. Kompressionssystem nach Anspruch 1, wobei jede der Innen- und Außenoberflächen (4, 3) von mindestens dem zweiten lateralen Seitenbereich (15) der Hülle eine Struktur aufweist oder mit einer Struktur versehen ist, die angepasst ist, um durch mechanische Befestigungselemente (61, 62), die auf der Innenoberfläche der inneren bzw. äußeren Klappe (51, 52) bereitgestellt sind, ergriffen zu werden.

3. Kompressionssystem nach einem der vorstehenden Ansprüche, wobei die mechanischen Befestigungselemente (61, 62), die auf den Innenoberflächen der Klappen (51, 52) bereitgestellt sind, Steckerbefestigungselemente (25, 27) umfassen, insbesondere Steckerbefestigungselemente ausgewählt aus der Gruppe bestehend aus Häkchenverschlüssen, pilzförmigen Befestigungselementen, stielförmigen Befestigungselementen, becherförmigen Befestigungselementen, T-förmigen Befestigungselementen, stiftförmigen Befestigungselementen und Mischungen davon.

4. Kompressionssystem nach einem der vorstehenden Ansprüche, wobei das Verschlusselement (5) ferner ein Sperrelement (53) oder eine Reihe von Sperrelementen (53) umfasst, wobei das Sperrelement bzw. die Sperrelemente zusammen mit der äußeren Klappe (51) oder den äußeren Klappen (51), wie zutreffend, bereitgestellt ist bzw. sind, wobei das/die Sperrelement(e) und die äußere(n) Klappe(n) derart konfiguriert und angeordnet sind, dass bei Verwendung, wenn das Verschlusselement (5) an der Hülle (1) befestigt ist, das/die Sperrelement(e) (53) an die äußere(n) Klappe(n) (51) geklammert werden kann/können, insbesondere auf die Außenoberfläche der äußeren Klappe(n) (51).

5. Kompressionssystem nach einem der vorstehenden Ansprüche, wobei das Verschlusselement (5) eine oder mehrere längliche Versteifungen (42) einschließt, die in Längsrichtung verlaufen.

6. Kompressionssystem nach einem der vorstehenden Ansprüche, wobei das Verschlusselement (5) einen Abstandshalterstoff und/oder einen Schaumstoff umfasst.

7. Kompressionssystem nach einem der vorstehenden Ansprüche, wobei jedes Befestigungsband (2) einen distalen Endabschnitt (24) umfasst, wobei jedes Band (2) im Wesentlichen in der Querrichtung der Hülle (1) verläuft, wobei sein distaler Endabschnitt (24) vom zentralen Abschnitt (14) der Hülle weg positioniert ist.

8. Kompressionssystem nach Anspruch 6, wobei eine Außenoberfläche des sekundären Verschlusselements (5) eine Struktur aufweist oder mit einer Struktur (55) versehen ist, die angepasst ist, um durch mechanische Befestigungselemente (56) auf der ersten Hauptoberfläche (44) des distalen Endabschnitts (24) der Befestigungsbänder (2) ergriffen zu werden und wobei das Paar (50) Klappen (51, 52) oder die Paare (50) Klappen (51, 52), wie zutreffend, des Verschlusselements (5) in der Längsrichtung zu einer Seite der eingreifenden Struktur (55) für die Befestigungsbänder (2) verläuft bzw. verlaufen.

9. Kompressionssystem nach einem der Ansprüche 1 bis 6, wobei das sekundäre Verschlusselement (5) mit einer Mehrzahl von Ringen (16) in einer Reihe derart bereitgestellt ist, dass, wenn das Verschlusselement (5) an der Hülle (1) befestigt ist, jeder Ring (16) sich gegenüber eines Befestigungsbands (2) befindet, und wobei das Paar (50) Klappen (51, 52) oder die Paare (50) Klappen (51, 52), wie zutreffend, in Längsrichtung zu einer Seite der Reihe von Ringen (16) verläuft bzw. verlaufen.

## Revendications

1. Système de compression (100) pour appliquer une compression à un membre d'un utilisateur comprenant un manchon (1) pour couvrir sensiblement une partie du membre d'un utilisateur,
dans lequel le manchon a une surface externe (3), une surface interne (4), un bord supérieur (7), un bord inférieur (8) et deux bords latéraux (9, 10), dans lequel, dans la direction transversale allant du premier bord latéral au deuxième bord latéral, le manchon comprend une première région latérale (13), une région centrale (14) et une deuxième région latérale (15).
la première région latérale (13) du manchon étant pourvue d'une pluralité de bandes de fixation (2) en série entre les bords supérieur et inférieur du manchon et la deuxième région latérale (15) étant ajustable,
et dans lequel le système comprend en outre un élément de fermeture secondaire (5), ledit élément de fermeture pouvant être fixé de façon libérable à la deuxième région latérale (15), ledit élément de fermeture étant en outre pourvu soit d'une paire allongée unique (50) de rabats s'étendant longitudinalement soit d'une pluralité de paires (50) de rabats (51, 52) s'étendant longitudinalement en série, ladite paire allongée de rabats ou lesdites paires de rabats, selon ce qui est applicable, incluant chacune un rabat extérieur (51) recouvrant un rabat intérieur (52), dans lequel au moins le rabat extérieur peut être déplacé par rapport au rabat intérieur de telle sorte que le rabat extérieur est susceptible d'effectuer un mouvement de charnière autour d'un axe longitudinal ou essentiellement longitudinal (L) et par rapport au rabat intérieur et dans lequel les surfaces internes opposées des rabats intérieur et extérieur comprennent des éléments de fixation mécanique (61, 62) conçus pour venir en prise de manière libérable avec les surfaces interne et externe (4, 3), respectivement, d'au moins la deuxième région latérale (15) du manchon et pour fixer l'élément de fermeture secondaire le long de la deuxième région latérale du manchon à la manière d'un collier de serrage.

2. Système de compression selon la revendication 1, dans lequel chacune des surfaces interne et externe (4, 3) d'au moins la deuxième région latérale (15) du manchon a une structure ou est pourvue d'une structure qui est conçue pour être mise en prise par des éléments de fixation mécanique (61, 62) fournis sur la surface interne des rabats intérieur et extérieur (51, 52), respectivement.

3. Système de compression selon l'une quelconque des revendications précédentes, dans lequel les éléments de fixation mécanique (61, 62) fournis sur les surfaces internes des rabats (51, 52) comprennent des éléments de fixation mâles (25, 27) en particulier des éléments de fixation mâles choisis dans le groupe constitué d'éléments de fixation à crochets, éléments de fixation en forme de champignon, éléments de fixation en forme de tige, éléments de fixation en forme de coupelle, éléments de fixation en forme de T, éléments de fixation en forme de broche et des mélanges de ceux-ci.

4. Système de compression selon l'une quelconque des revendications précédentes, dans lequel l'élément de fermeture (5) comprend en outre un élément de verrouillage (53) ou une série d'éléments de verrouillage (53), ledit ou lesdits élément(s) de verrouillage étant fournis en association avec le rabat extérieur (51) ou les rabats extérieurs (51), selon ce qui est applicable, ledit ou lesdits élément(s) de verrouillage et rabat(s) extérieur(s) étant configuré(s) et agencé(s) de telle sorte que, à l'usage, lorsque l'élément de fermeture (5) est fixé au manchon (1), le(s) élément(s) de verrouillage (53) peu(ven)t être pincé(s) sur le(s) rabat(s) extérieur(s) (51), en particulier sur la surface externe du ou des rabat(s) extérieur(s) (51).

5. Système de compression selon l'une quelconque des revendications précédentes, dans lequel l'élément de fermeture (5) inclut un ou plusieurs raidisseurs allongés (42) s'étendant longitudinalement.

6. Système de compression selon l'une quelconque des revendications précédentes, dans lequel l'élément de fermeture (5) comprend un tissu d'espacement et/ou une mousse.

7. Système de compression selon l'une quelconque des revendications précédentes, dans lequel chaque bande de fixation (2) comprend une partie d'extrémité distale (24), chaque bande (2) s'étendant essentiellement dans la direction transversale du manchon (1) avec sa partie d'extrémité distale (24) positionnée à l'écart de la partie centrale (14) du manchon.

8. Système de compression selon la revendication 6, dans lequel une surface externe de l'élément de fermeture secondaire (5) a une structure ou est pourvue d'une structure (55) qui est conçue pour être mise en prise par les éléments de fixation mécanique (56) sur la première surface principale (44) de la partie d'extrémité distale (24) des bandes de fixation (2) et dans lequel ladite paire (50) de rabats (51, 52) ou lesdites paires (50) de rabats (51, 52), selon ce qui est applicable, de l'élément de fermeture (5) s'étendent longitudinalement vers un côté de ladite structure de mise en prise (55) pour les bandes de fixation (2).

9. Système de compression selon l'une quelconque des revendications 1 à 6, dans lequel l'élément de fermeture secondaire (5) est pourvu d'une pluralité d'anneaux (16) en série de telle sorte que, lorsque l'élément de fermeture (5) est fixé au manchon (1), chaque anneau (16) est situé opposé à une bande de fixation (2), et dans lequel ladite paire (50) de rabats (51, 52) ou lesdites paires (50) de rabats (51, 52), selon ce qui est applicable, s'étendent longitudinalement vers un côté de la série d'anneaux (16).
